# FASCICULE DE BREVET EUROPEEN

(11) **EP 3 664 769 B1**
(45) Date de publication et mention de la délivrance du brevet: **26.07.2023**
(21) Numéro de dépôt: 18765485.0
(22) Date de dépôt: 09.08.2018
(51) Int. Cl.: A61K 8/06, A61Q 19/10, A61Q 5/02, A61K 8/44, A61K 8/46

(54) **EMULSION MOUSSANTE A HAUTE TENEUR EN HUILE ET SES UTILISATIONS, NOTAMMENT POUR L'HYGIENE CORPORELLE**
SCHÄUMENDE EMULSION MIT HOHEM ÖLGEHALT UND IHRE VERWENDUNG, INSBESONDERE FÜR DIE KÖRPERHYGIENE
HIGH OIL-CONTENT FOAMING EMULSION AND USES THEREOF, IN PARTICULAR FOR PERSONAL HYGIENE

(30) Priorité: 11.08.2017 FR 1757660
(43) Date de publication de la demande: 17.06.2020
(73) Titulaire: Laboratoires De Biologie Vegetale Yves Rocher, 56200 La Gacilly (FR)
(72) Inventeur: LAURENT, Marie-Agnès, 91220 Brétigny-Sur-Orge (FR); BOUVET, Estelle, 72000 Le Mans (FR)
(74) Mandataire: Novagraaf Technologies
(86) Numéro de dépôt international: PCT/FR2018/052039
(87) Numéro de publication internationale: WO 2019/030458

(56) Documents cités:
- EP-A1- 3 175 838
- US-A1- 2010 307 523
- US-A1- 2015 297 489

## Description

### Domaine technique

La présente invention se rapporte à une émulsion moussante comprenant au moins un tensio-actif anionique, au moins un composé choisi parmi les tensio-actifs amphotères et les tensio-actifs non-ioniques et une phase huile.

La présente invention trouve des applications notamment dans le domaine de la cosmétique.

### Etat de la technique

Au cours des trente dernières années, l'utilisation des gels douche est progressivement devenue un geste quotidien lors de la toilette, du fait de la sensation agréable qu'ils procurent sur la peau, de leurs parfums variés et de leur praticité d'utilisation.

Les crèmes de douche sont une alternative aux gels douche pour les personnes ayant une peau sensible, sèche ou recherchant plus de richesse et de douceur lors de l'application du produit et en termes de résultat sur la peau.

Les crèmes de douche sont en effet sensées contenir des agents de soin pour la peau. Cependant, la formulation de ces agents de soin, comme les huiles, est souvent difficilement compatible avec l'obtention d'une mousse suffisamment abondante, généralement attendue pour produits de douche. Ainsi, dans les huiles de douche, le pouvoir moussant n'est généralement pas satisfaisant.

Pour pouvoir obtenir un produit suffisamment moussant, la plupart des crèmes de douche actuellement sur le marché sont formulées sur la même base qu'un gel douche, enrichi en agents adoucissants et opacifié par ajout d'agents opacifiants. Dans ces formules, l'agent opacifiant le plus utilisé est le styrène acrylate copolymer, un polymère synthétique qui présente l'inconvénient de ne pas être facilement biodégradable. Les agents adoucissants sont quant à eux des polymères cationiques, non facilement biodégradables et écotoxiques. Enfin, la suspension de l'agent opacifiant nécessite fréquemment l'ajout d'un polymère synthétique gélifiant pour assurer la stabilité de la formulation dans le temps. Les gélifiants les plus utilisés sont les polymères acryliques qui ne sont pas facilement biodégradables. Le gélifiant peut également permettre d'augmenter la viscosité, ce qui contribue à la richesse du produit pour le consommateur mais présente l'inconvénient de ralentir la dilution de la crème de douche à l'eau et de ce fait le départ de mousse.

Le document EP3175838 décrit des compositions H/E contenant au maximum 0,76% de matière active en poids par rapport à leur poids total, comprenant :
- le disodium laureth sulfosuccinate comme tensio-actif anionique,
- le capryl/capramidopropyl bétaïne comme tensio-actif amphotère,
- le diéthylhexyl carbonate comme un émollient, et
- le Bis-PEG/PPG-20/5 PEG/PPG-20/5 Dimethicone; Methoxy PEG/PPG-25/4 Dimethicone; caprylic/capric triglycérides comme émulsifiant silicone H / E.

Il est possible de trouver sur le marché de vraies émulsions de douche basées sur des bases moussantes structurées en phases lamellaires, mais leur teneur en huile reste modérée. De plus, ces formules contiennent généralement des conservateurs qui ne sont pas neutres d'un point de vue innocuité et d'un point de vue environnemental. Ces formules contiennent également un fort pourcentage de sel, utilisé pour la création des phases lamellaires, ce qui peut être un facteur d'irritation de la peau. Enfin, ces formules contiennent généralement des tensio-actifs, éthoxylés, peu satisfaisants du point de vue écologique, puisque l'éthoxylation est une réaction chimique présentant des risques environnementaux de par l'utilisation d'oxyde d'éthylène, hautement réactif et toxique. La production de tensio-actifs éthoxylés nécessite d'ailleurs l'installation de sites de production classés à haut risque environnemental.

Ainsi, il existe un réel besoin de produits d'hygiène corporel palliant ces défauts, inconvénients et obstacles de l'art antérieur, en particulier de produits de type crèmes de douche formulés à partir d'ingrédients d'origine végétale, facilement biodégradables et non écotoxiques, apportant du soin tout en présentant une mousse abondante lors de l'utilisation.

### Description de l'invention

Aux termes d'importantes recherches, la Demanderesse est parvenue à mettre au point une émulsion moussante concentrée en huiles répondant précisément à ces besoins.

L'émulsion, objet de l'invention peut en effet contenir plus de 50% d'huile, ce qui lui confère un effet nourrissant et adoucissant important, tout en produisant une mousse agréable lors de son utilisation, comparable à celle formée par une crème de douche classique. De manière surprenante, de par sa forte concentration en tensio-actifs et en huile, l'émulsion de l'invention ne contient aucun conservateur, ce qui confère un bénéfice d'innocuité pour le consommateur, et réduit l'impact du produit sur l'environnement par rapport aux produits classiques.

De manière avantageuse, l'aspect blanc de l'émulsion de l'invention est uniquement lié à sa structure d'émulsion, ce qui permet d'éviter l'ajout d'agents opacifiants. En outre, l'émulsion de l'invention présente la particularité de blanchir lorsqu'elle est diluée dans l'eau, prenant alors l'apparence agréable d'une eau lactée, ce qui renforce encore la perception de l'effet de soin par l'utilisateur.

Par ailleurs, les agents moussants de l'émulsion de l'invention peuvent être des tensio-actifs non-éthoxylés, notamment pouvant présenter une bonne « naturalité », voire être autorisés dans les produits moussants bio. La crème de douche peut notamment être 100% d'origine naturelle si l'on intègre un parfum d'origine naturelle ou si elle est formulée sans parfum.

L'émulsion objet de l'invention présente donc l'avantage de ne pas inclure de conservateur, d'agent opacifiant, de polymère acrylique gélifiant ou stabilisant ni aucun polymère cationique pour adoucir la peau, tout en contenant des ingrédients d'origine naturelle, ne présentant pas les inconvénients pour l'environnement des additifs des crèmes de douche classiques.

Ainsi, un premier objet de l'invention se rapporte à une émulsion huile dans l'eau moussante comprenant :
a) au moins un tensio-actif anionique,
b) au moins un composé choisi parmi les tensio-actifs amphotères et les tensio-actif non-ioniques, et
c) au moins une phase huile,
   - dans laquelle la matière active totale desdits tensio-actifs est comprise de 4,0 à 30% en poids par rapport au poids total de la dite émulsion,
   - dans laquelle le ratio de tensio-actif anionique est compris de 0,3 à 0,9, le ratio de tensio-actif amphotère est compris de 0,0 à 0,7 et le ratio de tensio-actif non-ionique est compris de 0,0 à 0,7, ledit ratio étant le rapport entre le pourcentage en poids de matière active dudit tensio-actif dans ladite composition et le pourcentage en poids de matière active totale desdits tensio-actifs dans ladite émulsion,
   - dans laquelle ladite phase huile est comprise de 50 à 98 % en poids du poids total de l'émulsion, 50% étant exclu,
   - le pH de l'émulsion étant compris entre 4 et 8,
caractérisée en ce que :
ledit au moins un tensio-actif anionique est choisi parmi :
   o les alkyl sulfates,
   o les alkyl éther sulfates choisis dans le groupe le sodium trideceth sulfate, l'ammonium laureth sulfate, le magnésium laureth sulfate et le triethanolamine laureth sulfate, le diethanolamine laurethsulfate, le monoethanolamine laurethsulfate, et leurs mélanges,
   o les sulfonates,
   o les phosphates et les alkyl phosphates,
   o les iséthionates,
   o les taurates,
   o les alkyl sulfoacetates,
   o les dérivés anioniques de protéines d'origine végétale,
   o les dérivés des aminoacides,
   o les acides carboxyliques et leurs sels,
   o les sels d'acides gras issus de la saponification
   o les dérivés d'acide lactique,
   o les dérivés d'alkyl polyglucoside, et
   o les sulfosuccinates choisis dans le groupe comprenant le disodium undecylenamido MEA- sulfosuccinate, le disodium lauryl sulfosuccinate, le disodium ricinoleamido MEA-sulfosuccinate, et leurs mélanges,
le composé b) comprenant un tensio-actif amphotère.

On entend par « matière active », au sens de la présente invention, le tensio-actif présent dans la matière première commerciale contenant le tensio-actif. La matière active est ainsi à l'origine des propriétés tensio-actives de la matière première contenant le tensio-actif. La matière première, généralement distribuée par un fournisseur, contient, en plus de la matière active ayant les propriétés tensio-actives, d'autres composants, comme par exemple de l'eau ou des solvants, des résidus réactionnels et des produits annexes. Le pourcentage de matière active dans une matière première s'obtient en soustrayant le pourcentage d'eau ou solvant, les résidus réactionnels et les produits annexes contenus dans la matière première pour ne compter que le pourcentage du tensio-actif.

On entend par « matière active totale », au sens de la présente invention, la somme des matières actives présentes dans chaque matière première contenant chaque tensio-actif, et comprises dans l'émulsion objet de l'invention.

Selon l'invention, la matière active totale des tensio-actifs est comprise de 4,0 à 30% en poids par rapport au poids total de l'émulsion, par exemple de 4,0 à 20,0, ou de 5,0 à 25,0%, ou de 10,0 à 20,0%, ou de 20,0 à 30,0%.

L'émulsion objet de l'invention a un pH compris de 4,0 à 8,0, les bornes étant incluses. Le pH peut être par exemple compris de 4,5 à 7,5, ou entre 5,0 et 7,0, ou entre 5,5 et 6,5, les bornes étant incluses. Avantageusement, le pH peut être un pH physiologique compris entre 5,0 et 6,0, les bornes étant incluses.

Selon l'invention, la phase huile est comprise de 50 à 98 % en poids du poids total de la composition, 50% n'étant pas inclus. Il peut s'agir par exemple de 55 à 98%, ou de 60 à 98%, ou de 70 à 98%, ou de 80 à 98%. La phase huile peut être au moins une huile naturelle, et/ou au moins une huile de synthèse, et/ou au moins un beurre ainsi que leurs mélanges.

On entend par « huile naturelle », au sens de la présente invention, une huile extraite uniquement par des procédés mécaniques de graines ou de fruits. Comme exemple d'huile naturelle, on peut citer toute huile adaptée, comme les triglycérides naturels par exemple les huiles d'avocat, de sésame, de tournesol, de colza, de maïs, de soja, de carthame, de pépins de raisin, d'arachide, de ricin, de coco (ou coprah), de graines de coton, d'olive, de palmiste, de son de riz, d'amande douce, de canola, d'abricot, de noix et de germe de blé, ou comme les cires liquides par exemple, l'huile de jojoba ou l'huile de lanoline.

Comme exemple d'huile de synthèse, on peut citer toute huile d'origine naturelle ou pétrochimique adaptée, comme des alcools, des alcanes, des alcènes, des esters, des éthers, des huiles silicones ou des huiles fluorées, cette liste n'étant pas limitative. Parmi les alcools on peut citer les alcools gras liquides comme l'alcool isocétylique ou l'octyldodecanol. Parmi les alcanes liquides on peut citer le C11 C12 isoalcane (fourni par exemple sous la marque CETIOL^{®} ULTIMATE de Basf) ou des alcanes linéaires de C12 à C22 (fourni par exemple sous la marque EMOGREEN^{™} de Seppic ou PARAFOL^{®} de Sasol), les huiles de paraffine, le C11 C12 isoparaffin, l'isohexadécane, l'isoéicosane, l'isododécane, le squalane ou l'hémisqualane. Parmi les alcènes on peut citer le squalène. Parmi les esters, on peut citer par exemple, le myristate d'isopropyle, le cococaprylate caprate, le palmitate d'ethylhexyle, les alkyl benzoate, le triglycéride C8 C10. Parmi les éthers on peut citer par exemple le dicaprylyl ether. Parmi les huiles silicone on peut citer les huiles de silicone volatiles ou non volatiles telles que les polydiméthylsiloxanes (PDMS), les cyclodiméthylsiloxanes ou cyclométhicones. Parmi les huiles fluorées ou fluorosiliconées, on peut citer le polyperfluoromethylisopropyl ether, par exemple faisant partie de la gamme des FOMBLIN^{®} de Solvay.

Les beurres, également appelés corps gras pâteux, c'est-à-dire les corps gras ayant un point de fusion proche de la température cutané 37°C, peuvent également être utilisés seuls ou en association avec les huiles citées ci-dessus. Comme exemple de corps gras pâteux naturels, on peut citer le beurre de karité, le beurre de cacao, le beurre de coco, l'huile de palme et la lanoline qui est un corps gras pâteux d'origine animale. Comme beurres ou corps gras pâteux synthétiques on peut citer les dérivés de lanoline, les huiles hydrogénées, les esters comme le myristyl myristate, le myristyl lactate, le bis-diglyceryl polyacyladipate-2 ou l'arachidyl propionate, les cires silicones comme par exemple le DC AMS-C30 de Dow Corning^{®} (C30-45 alkyl methicone & C30-45 olefin) ou des élastomères silicone comme le DC EL-9240 de Dow Corning^{®} (dimethicone & dimethicone crosspolymer), les éthers, par exemple le PEG-5 pentaérythrityl éther & PPG-5 pentaérythrityl éther & glycine soja stérols comme le LANOLIDE 02.0911 de Vevy Europe, des polyglycérides comme les polyglycérides oléiques, linoléniques et hydroxystéariques VIAMERINES^{®} de Aldivia, et les alcanes comme la vaseline.

Des corps gras solides naturels ou synthétiques peuvent être ajoutés aux huiles et aux beurres. On entend par « corps gras solide », au sens de la présente invention, tout corps gras ayant un point de fusion supérieur à 40°C. Ils peuvent inclure les cires naturelles, parmi lesquelles on peut citer la cire d'abeille, la cire de carnauba, la cire de sumac ou la cire de candelilla, les alcools gras solides de 14 à 36 carbones, par exemple les alcools myristique, cétylique, stéarique, béhénique, les acides gras solides de 14 à 36 carbones par exemple acide myristique, cétylique, stéarique, hydroxystéarique ou béhénique, les huiles végétales hydrogénées comme l'huile de ricin hydrogénée ou la cire de riz hydrogénée, les esters avec par exemple le cétyl ricinoléate ou le cétylpalmitate, les composés hydrocarbonés de 20 à 50 carbones, par exemple la cire de polyéthylène, la paraffine, l'ozokérite, la cire microcristalline et plus largement tous les corps gras solides appartenant aux catégories chimiques décrites pour les huiles mais présentant un point de fusion supérieur à 40°C.

Avantageusement, cette forte quantité d'huile ou de phase huile/grasse peut permettre de limiter ou de supprimer tout autre agent de soin, ou tout agent adoucissant. En effet, il est possible, dans certains modes de réalisation de l'invention, que tout le bénéfice soin soit apporté par l'huile ou la phase huile/grasse.

Avantageusement, l'émulsion objet de l'invention présente un bon pouvoir moussant, c'est-à-dire une capacité à former suffisamment rapidement une mousse onctueuse sur la peau lors de son application sur une peau ou des cheveux mouillés. Le pouvoir moussant peut être mesuré par toute méthode adaptée connue de l'homme du métier, par exemple par un test d'usage par des consommateurs habitués à l'utilisation des crèmes de douche classiques.

De manière particulièrement avantageuse, la composition de l'invention peut être dépourvue de conservateur. En d'autres termes, la composition ne contient pas d'agent de conservation autre que les composants expressément mentionnés comme susceptibles de faire partie de la composition de l'invention, par exemple les tensio-actifs, huiles et/ou parfum, qui pourraient présenter intrinsèquement des propriétés de conservation de la composition de l'invention. La composition de l'invention présente en effet l'avantage d'avoir, sans ajout de conservateur dédié, une stabilité dans le temps comparable à celle des produits classiques, notamment ceux comprenant des conservateurs. La composition de l'invention présente en outre l'avantage d'éviter les risques liés à l'ajout de conservateurs, comme les perturbations du système hormonal, les irritations cutanées, ou les allergies cutanées. A titre d'exemple, les conservateurs non utilisés dans le cadre de la composition de l'invention sont les parabènes, le triclosan, le méthylisothiazolinone, les libérateurs de formols / de formaldéhyde, le phénoxyéthanol, le benzoate de sodium, l'acide salicylique et l'acide sorbique, cette liste n'étant pas limitative.

L'eau peut être comprise d'environ 1 à 40% en poids par rapport au poids total de la composition de l'invention. Par exemple, il peut s'agir de 5 à 30%.

Selon l'invention, la composition de l'invention comprend au moins un composé choisi parmi les tensio-actifs amphotères et les tensio-actifs non-ioniques. Ainsi, lorsque le ratio de tensio-actif amphotère est de 0,0, le ratio de tensio-actif non-ionique ne peut être égal à 0,0, la valeur de 0,0 est donc exclue dans ce cas. Inversement, lorsque le ratio de tensio-actif non-ionique est égal à 0,0, le ratio de tensio-actif amphotère ne peut être égal à 0,0, la valeur de 0,0 est donc exclue dans ce cas.

Selon l'invention, le ratio de tensio-actif anionique est compris de 0,3 à 0,9, par exemple de 0,4 à 0,8, ou de 0,5 à 0,7.

Selon l'invention, le ratio de tensio-actif amphotère est compris de 0,0 à 0,7, par exemple de 0,1 à 0,6, ou de 0,2 à 0,5.

Selon l'invention, le ratio de tensio-actif non-ionique est compris de 0,0 à 0,7, par exemple de 0,1 à 0,6, ou de 0,2 à 0,5.

Par exemple, une composition de l'invention peut consister en la composition telle que décrite ci-avant.

Dans ce cas, aucun autre composé que ceux indiqués dans cet exemple n'est ajouté dans la composition de l'invention.

Selon l'invention, les ratios et pourcentages indiqués pour la composition de l'invention peuvent être mesurés par toute méthode connue de l'homme du métier. Il peut s'agir par exemple, notamment pour le dosage des tensio-actifs anioniques, de la norme NF ISO 2271 (Détermination de la teneur en matières actives anioniques selon une méthode manuelle ou mécanique par titrage direct dans deux phases, janvier 1990, AFNOR).

Selon l'invention,
- les alkyl sulfates peuvent être choisis dans le groupe comprenant l'ammonium lauryl sulfate, le sodium lauryl sulfate, le sodium coco sulfate, le potassium laurylsulfate, le magnésium laurylsulfate, le triethanolamine laurylsulfate, le diethanolamine laurylsulfate, le monoethanolamine laurylsulfate et leurs mélanges ;
   - les sulfonates peuvent être choisis dans le groupe comprenant le sodium C14-16 olefin sulfonate, le sodium C14-17 sec-Alkyl sulfonate et le sodium xylene sulfonate et leurs mélanges ;
   - les phosphates et alkyl phosphates peuvent être choisis dans le groupe comprenant le dicetyl phosphate, le C12-15 phosphate, le Potassium cetyl phosphate et le C9-15 alkyl phosphate et leurs mélanges ;
   - les iséthionates peuvent être choisis dans le groupe comprenant le sodium cocoyl isethionate et le sodium Lauroyl Methyl Isethionate et leurs mélanges ;
   - les taurates peuvent être choisis dans le groupe comprenant le sodium methyl cocoyl taurate et le sodium stearoyl taurate et leurs mélanges ;
   - les alkyl sulfoacetates peuvent être le sodium lauryl sulfoacetate ;
   - les dérivés anioniques de protéines d'origine végétale peuvent être choisis parmi le sodium cocoyl apple amino acids, le sodium lauroyl oat amino acids, le sodium lauroyl wheat amino acids et le potassium lauroyl wheat amino acids et leurs mélanges ;
   - les aminoacides et dérivés des aminoacides peuvent être choisis parmi le sodium lauroyl glutamate, le sodium cocoyl glutamate,
   le potassium cocoyl glycinate, le sodium cocoyl alaninate et le sodium lauroyl sarcosinate et leurs mélanges ;
   - les acides carboxyliques et leurs sels peuvent être choisis dans le groupe comprenant le sodium lauryl glucose carboxylate et le sodium laureth-13 carboxylate et leurs mélanges ;
   - les sels d'acides gras issus de la saponification peuvent être choisis parmi les sels de l'acide laurique, l'acide myristique, l'acide palmitique et l'acide stéarique et leurs mélanges ;
   - les dérivés d'acide lactique peuvent être choisis parmi le sodium stéaroyl lactylate, le sodium isostéaryl lactylate et le sodium cocoyl lactylate et leurs mélanges ;
   - les dérivés d'alkyl polyglucoside peuvent être choisis parmi le sodium cocoglucoside tartrate, le disodium cocopolyglucose sulfosuccinate et le disodium cocopolyglucose citrate et leurs mélanges.

De préférence, le tensio-actif anionique majoritaire de la composition de l'invention est le laurylsulfate d'ammonium, un tensio-actif non éthoxylé qui présente une bonne « naturalité », et qui est autorisé dans les produits moussants bio.

Selon l'invention, l'au moins un tensio-actif amphotère peut être choisi dans le groupe comprenant les N-alkylamidobétaines, les bétaines, les sultaines, les alkylpolyaminocarboxylates, les alkylamphoacétates, leurs dérivés, et les dérivés de la glycine.

Selon l'invention :
- les N-alkylamidobétaines peuvent être choisies dans le groupe comprenant la cocamidopropyl bétaine et la lauramidopropyl bétaine et leurs mélanges ;
- les bétaines peuvent être choisies dans le groupe comprenant la coco bétaine et la lauryl bétaine et leurs mélanges ;
- les sultaines peuvent être la cocamidopropyl hydroxysultaine,
- les alkylpolyaminocarboxylates peuvent être choisis dans le groupe comprenant le sodium Carboxymethyl Tallow Polypropylamine et le sodium Carboxymethyl Oleyl Polypropylamine et leurs mélanges;
- les alkylamphoacétates peuvent être choisis dans le groupe comprenant le disodium cocoamphoacetate, le sodium cocoamphoacetate et le disodium lauroamphoacetate et leurs mélanges ;
- les dérivés de la glycine peuvent être le cocoamphopolycarboxyglycinate.

Selon l'invention, au moins un tensio-actif non-ionique peut être choisi dans le groupe comprenant les alkypolyglucosides, les esters de glycéryle et d'acide gras, les esters de sucrose et d'acides gras, les esters de sucrose oxyalkylénés, les esters de glycérol oxyalkylénés, les esters d'acides gras et de polyéthylène glycol, les esters d'acide gras et de sorbitan, les alcools gras polyglycérolés et les dérivés de glucamine.

Selon l'invention :
- les alkypolyglucosides peuvent être choisis dans le groupe comprenant le décyl glucoside, le lauryl glucoside, le caprylyl/capryl glucoside, et le coco glucoside, et leurs mélanges;
- les esters de glycéryle et d'acide gras peuvent être choisis parmi le glycéryl stéarate, le glycéryl ricinoléate le glycéryl oléate, le glycéryl caprylate, le glycéryl caprate et leurs mélanges ;
- les esters de sucrose et d'acides gras peuvent être choisis parmi le sucrose stéarate, le sucrose palmitate, le sucrose laurate et le sucrose distearate, et leurs mélanges ;
- les esters de sucrose oxyalkylénés peuvent être choisis parmi le méthyl glucose caprate/caprylate/oleate, le PEG-120 Methyl glucose dioleate et le PEG-20 Methyl glucose sesquistearate, et leurs mélanges ;
- les esters de glycérol oxyalkylénés peuvent être choisis parmi le PEG-7 Glyceryl cocoate, le PEG-80 Glyceryl cocoate, le PEG-30 Glyceryl cocoate et le PEG-200 hydrogenated glyceryl palmate, et leurs mélanges ;
- les esters d'acides gras et de polyéthylène glycol peuvent être choisis dans le groupe le PEG-8 stéarate, le PEG-20 stéarate, le PEG-40 stéarate, le PEG-50 stéarate et le PEG-100 stéarate, et leurs mélanges ;
- les esters d'acide gras et de sorbitan peuvent être choisis dans le groupe comprenant le sorbitan palmitate, le sorbitan stéarate, le sorbitan tristéarate, le sorbitan oléate et le sorbitan trioléate, et leurs mélanges ;
- les dérivés de glucamine peuvent être choisis dans le groupe comprenant le capryloyl ou le caproyl méthyl glucamide, le lauroyl méthyl Glucamide, le lauroyl méthyl glucamide et le cocoyl méthyl glucamide, et leurs mélanges.

Avantageusement, le tensio-actif anionique peut être un alkyl sulfate, le tensio-actif amphotère peut être une N-alkylamidobétaïne ou un alkylamphoacétate, et le tensio-actif non-ionique peut être un alkypolyglucoside.

Avantageusement, le tensio-actif anionique peut être choisi parmi l'ammonium lauryl sulfate, le sodium methyl cocoyl taurate et le sodium laurylsarcosinate ; le tensio-actif amphotère peut être la cocamidopropylbétaine ou le cocoamphoacétate de sodium, et le tensio-actif non-ionique peut être le décylglucoside ou le glycéryl oléate.

La composition de l'invention peut en outre comprendre au moins un parfum. On entend par « parfum », au sens de la présente invention, une matière première parfumante ou un mélange de matières premières parfumantes, également nommé « concentré parfum », d'origine naturelle et/ou synthétique, solubilisées ou non dans un solvant pour matières premières de parfumerie. En d'autres termes, il peut s'agir de toute composition odorante ou de tout mélange de matières premières odorant, présentant des caractéristiques olfactives compatibles avec une utilisation en cosmétique, généralement fortement concentrée, généralement proposée conditionnée par un parfumeur. Le solvant peut être tout solvant adapté connu de l'homme du métier, comme par exemple l'éthanol, le Dipropylene glycol, le triéthylcitrate, le myristate d'isopropyl, la triacétine, cette liste n'étant pas limitative. Les matières premières d'origine naturelle peuvent être toute matière première adaptée connue de l'homme du métier, par exemple les huiles essentielles, les concrètes, les absolus ou les extraits végétaux. Les proportions entre solvant et matières premières odorantes peuvent être très variables selon la puissance olfactive des matières premières odorantes et leur solubilité, selon ce qui est classiquement réalisé dans le domaine des parfums, connu de l'homme du métier. Selon l'invention, le pourcentage en poids de parfum dans la composition participe à l'agrément de la composition-Par exemple, le pourcentage de parfum peut aller de 0,00 à 10,00%, en poids par rapport au poids total de la composition, par exemple de 0,05 à 10,00 % ou de de 0,10 à 5,00%, ou de 0,50 à 1,00%. Avantageusement, le parfum, lorsqu'il est présent, peut participer à la création des phases lamellaires dans la phase aqueuse.

Avantageusement, la composition de l'invention peut ne pas contenir de chlorure de sodium autre que celui pouvant être apporté par les tensio-actifs. En effet, il n'est pas ajouté de chlorure de sodium en tant que tel lors de la préparation de la composition de l'invention. Toutefois, les compositions de tensio-actifs faisant parties de la composition peuvent en contenir, ce qui implique qu'une faible quantité de chlorure de sodium puisse éventuellement être détectée dans la composition de l'invention. Il peut s'agir par exemple d'environ 0,005 à environ 4,000% en poids de chlorure de sodium venant des compositions de tensio-actifs, par rapport au poids total de ladite composition, par exemple entre 0,050 et 3,000% ou entre 0,15 et 2,000%, ou strictement inférieur à 2,000%, ou strictement inférieur à 3,000%, ou strictement inférieur à 4,000%. L'absence d'ajout de chlorure de sodium confère de manière avantageuse une meilleure innocuité de la composition par rapport aux produits classiques, dans lesquels du chlorure de sodium est ajouté.
L'émulsion de l'invention peut être obtenue par tout procédé approprié connu de l'homme du métier pour la fabrication d'une émulsion Huile dans Eau. Il peut s'agir du mélange d'au moins une phase huile avec une composition de surfactant structurée en phases lamellaires, le pourcentage de ladite composition de surfactant étant compris de 2 à 50% en poids bornes incluses, par rapport au poids total de ladite émulsion, et le pourcentage de l'au moins une phase huile étant compris de 50 à 98% en poids, 50% étant exclu, par rapport au poids total de ladite émulsion, ladite composition de surfactant comprenant :
a) au moins un tensio-actif anionique, et
b) au moins un composé choisi parmi les tensio-actifs amphotères et les tensio-actif non-ioniques, et
   - dans laquelle la matière active totale desdits tensio-actifs est comprise de 20 à 60% en poids par rapport au poids total de la dite composition,
   - dans laquelle le ratio de tensio-actif anionique est compris de 0,3 à 0,9, le ratio de tensio-actif amphotère est compris de 0,0 à 0.7 et le ratio de tensio-actif non-ionique est compris de 0,0 à 0,7, ledit ratio étant le rapport entre le pourcentage en poids de matière active dudit tensio-actif dans ladite composition et le pourcentage en poids de matière active totale desdits tensio-actifs dans ladite composition,
   - le pH de la composition étant compris entre 4 et 8,
caractérisée en ce que :
ledit au moins un tensio-actif anionique est choisi parmi :
   o les alkyl sulfates,
   o les alkyl éther sulfates choisis dans le groupe le sodium trideceth sulfate, l'ammonium laureth sulfate, le magnésium laureth sulfate et le triethanolamine laureth sulfate, le diethanolamine laurethsulfate, le monoethanolamine laurethsulfate, et leurs mélanges,
   o les sulfonates,
   o les phosphates et les alkyl phosphates,
   o les iséthionates,
   o les taurates,
   o les alkyl sulfoacetates,
   o les dérivés anioniques de protéines d'origine végétale,
   o les dérivés des aminoacides,
   o les acides carboxyliques et leurs sels,
   o les sels d'acides gras issus de la saponification,
   o les dérivés d'acide lactique,
   o les dérivés d'alkyl polyglucoside, et
   o les sulfosuccinates choisis dans le groupe comprenant le disodium undecylenamido MEA- sulfosuccinate, le disodium lauryl sulfosuccinate, le disodium ricinoleamido MEA-sulfosuccinate, et leurs mélanges,
le composé b) comprenant un tensio-actif amphotère.

La composition de surfactant structurée en phases lamellaires peut en outre contenir de l'eau en quantité requise, que l'homme du métier saura adapter en fonction du pourcentage de tensio-actifs contenus dans la composition de surfactant.

Avantageusement, la composition de surfactant possède une structure en phases lamellaires, qui permet de stabiliser l'huile.

L'émulsion selon l'invention peut être une composition cosmétique ou dermatologique, notamment pour application cutanée, utilisée en particulier comme produit de nettoyage de la peau, y compris le corps et le visage, du cuir chevelu et/ou des cheveux.

Selon l'invention, la composition peut être sous une forme choisie parmi une crème de douche, une crème nettoyante pour la peau du visage, une crème démaquillante, un shampooing et une crème lavante pour les mains.

De préférence, la composition de l'invention est une crème de douche.

Selon l'invention, la composition de l'invention peut se présenter dans un conditionnement choisi parmi un flacon, par exemple de type un flacon muni d'une capsule ou d'une pompe, par exemple doseuse, un tube, par exemple un tube souple et un pot. Selon un mode de réalisation, la composition cosmétique ou dermatologique peut être constituée, c'est-à-dire comprendre exclusivement la composition de l'invention.

Alternativement, la composition cosmétique ou dermatologique peut comprendre en outre un véhicule cosmétiquement acceptable, et/ou physiologiquement acceptable, c'est-à-dire compatible avec la peau, les muqueuses, le cuir chevelu et/ou les cheveux.

On entend par « composition cosmétique », dans la présente invention, toute composition à visée cosmétique, c'est à dire esthétique, pouvant être mise en contact avec les parties superficielles du corps humain, par exemple l'épiderme, les systèmes pileux et capillaires, les organes externes et les muqueuses externes. Avantageusement, une composition cosmétique permet, exclusivement ou principalement de les nettoyer, les protéger ou les maintenir en bon état.

Dans la présente, on entend par « composition dermatologique » toute composition à visée dermatologique c'est à dire une composition pouvant être mise en contact avec les parties superficielles du corps humain, pour un traitement de la peau, des muqueuses et des phanères, ongles, cheveux, poils.

Par « véhicule cosmétiquement ou dermatologiquement acceptable », on entend un véhicule adapté pour une utilisation en contact avec des cellules humaines et animales cutanées, en particulier les cellules de l'épiderme, sans toxicité, irritation, réponse allergique indue et similaire, et proportionné à un rapport avantage/risque raisonnable. Le véhicule est utilisé dans des proportions compatibles avec la haute concentration de la composition de l'invention.

Selon l'invention, la composition cosmétique de l'invention peut comprendre en outre des adjuvants habituellement utilisés dans le domaine cosmétique, les actifs, les parfums, les conservateurs, les séquestrants (EDTA), les anti-oxydants, les polymères cationiques et tensio-actifs cationiques, les agents nacrants et opacifiants, les pigments, les charges minérales ou organiques telles que le talc, le kaolin, l'amidon, les exfoliants (particules solides végétales, minérales ou synthétiques ayant un effet abrasif sur la peau), les nacres et paillettes, les colorants, les filtres solaires, les acides et les bases pour ajustement du pH, les polymères synthétiques ou naturels gélifiants. Les quantités de ces différents adjuvants sont celles classiquement utilisées dans le domaine considéré, et par exemple de 0,01 à 20 % du poids total de la composition. Ces adjuvants ainsi que leurs concentrations doivent être tels qu'ils ne modifient pas la propriété recherchée pour la composition de l'invention.

Comme actifs, on peut citer par exemple les hydratants et par exemple les polyols tels que la glycérine, les glycols, les polyéthylène glycols et les sucres et leurs dérivés. On peut citer également les extraits naturels, la piroctone olamine, le zinc pyrithione, l'acide salicylique, l'urée, les vitamines et leurs dérivés (D panthenol ou acetate de tocopherol par exemple), les hydrolysats de protéines et les acides aminés.

La composition de l'invention peut être obtenue par tout procédé approprié connu de l'homme du métier pour la fabrication d'une composition cosmétique moussante. Il peut s'agir, par exemple d'un simple mélange.

Avantageusement, la composition peut être une composition à usage cutané.

Un autre objet de l'invention se rapporte à une utilisation cosmétique de la composition cosmétique selon l'invention, pour nettoyer la peau et/ou les phanères et/ou le cuir chevelu.

Un autre objet de l'invention se rapporte à un procédé de traitement cosmétique non thérapeutique comprenant l'application sur la peau et/ou des phanères et/ou le cuir chevelu d'une composition de l'invention ou d'une composition cosmétique selon l'invention.

Avantageusement, le procédé de traitement cosmétique peut être un procédé de nettoyage des résidus de salissure des matières kératiniques (également nettoyage de la sueur et des lipides) humaines, dans lequel la composition cosmétique de l'invention est appliquée sur la peau et/ou des phanères et/ou le cuir chevelu en présence d'eau. Avantageusement, l'application peut comprendre un massage pour former une mousse, qui peut être éliminée avec les résidus de salissure par rinçage à l'eau.

Dans le cadre des procédés cosmétiques selon l'invention, ou de l'utilisation selon l'invention, l'utilisation s'entend d'une utilisation non-thérapeutique, par exemple pour le traitement des peaux saines, c'est-à-dire des peaux ne présentant pas un état pathologique. Il peut également s'agir de peaux ne présentant pas de trace visible ou perceptible d'une agression extérieure, comme des démangeaisons, des coups de soleil, des brûlures, des piqûres, des signes d'inflammation, de plaie, cette liste n'étant pas limitative.

De préférence, toute utilisation cosmétique et tout procédé cosmétique selon l'invention sont respectivement des utilisations cosmétiques non-thérapeutiques et procédés cosmétiques non-thérapeutiques.

Le procédé de traitement de l'invention peut comprendre une étape de rinçage de l'émulsion après son application, en particulier lorsque l'émulsion est utilisée pour nettoyer la peau et/ou les phanères.

D'autres avantages pourront encore apparaître à l'homme du métier à la lecture des exemples ci-dessous, illustrés par les figures annexées, donnés à titre illustratif.

### EXEMPLES

### Exemple 1 : Exemples de compositions de l'invention

Des exemples de compositions, ainsi que leurs caractéristiques, sont décrites dans les tableaux I, II et III suivants.

**Tableau 1 :**

| | Matièr e active dans MP | HCSFE0 1 | HCSFE0 2 | HCSFB0 4 | HCSFB0 5 | HCSFB1 7 | HCSFB4 4 | HCSFB4 5 | HCSFB4 6 | HCSFB 47 | HCSFE 06 | HCSFE0 5 |
|---|---|---|---|---|---|---|---|---|---|---|---|---|
| Nom INCI | % MA dans TA | % MP commer ciale | % MP commer ciale | % MP commer ciale | % MP commer ciale | % MP commer ciale | % MP commer ciale | % MP commer ciale | % MP commer ciale | % MP comm erciale | % MP comm erciale | % MP commer ciale |
| Ammonium lauryl sulfate | 68 | 6,880 | 3,440 | 13,600 | 13,600 | 11,300 | 11,300 | 11,300 | 11,300 | 11,300 | 13,900 | 16,000 |
| Sodium methyl cocoyl taurate | 24 | 3,000 | | | | | | | | | | |
| Sodium laurylsarcosinate | 30 | | 1,500 | | | | | | | | | |
| cocamidopropylbetai ne | 35 | 4,600 | 2,300 | | 9,100 | 8,900 | 8,900 | 8,900 | 8,900 | 8,900 | 26,600 | 10,700 |
| cocoamphoacetate | 32 | | | 10,300 | | | | | | | | |
| decylglucoside | 53 | 0,860 | 0,430 | 1,700 | 1,700 | 2,900 | 2,900 | 2,900 | 2,900 | 2,900 | | 2,000 |
| glyceryl oleate | 90 | 0,170 | 0,090 | | | | | | | | | 0,400 |
| glyceryl oleate + cocoglucoside (LAMESOFT PO65 BASF) | 65 | | | | | 1,000 | 1,000 | 1,000 | 1,000 | 1,000 | | |
| acide citrique | | 0,048 | 0,024 | 0,750 | 0,050 | 0,060 | 0,060 | 0,060 | 0,060 | 0,060 | | 0,110 |
| parfum | | 0,490 | 0,240 | 0,800 | 0,800 | 0,750 | 0,750 | 0,750 | 0,750 | 0,750 | | 0,960 |
| gel aloe vera | | 0,190 | 0,100 | | | | | | | | | 0,440 |
| extrait grenade | | | | | | | | | | | | 0,200 |
| beurre Karité | | | | 1,000 | 1,000 | 64,000 | | | | | | |
| cococaprylate caprate | | | | | | | 64,000 | | | | | |
| isopropyl palmitate | | | | | | | | | | | | |
| undecane et tridecane | | | | | | | | 64,000 | | | | |
| squalane | | | | | | | | | 64,000 | | | |
| huile de colza | | | | | | | | | | 56,700 | | |
| huile de coprah | | | | | | | | | | 10,000 | | |
| huile tournesol | | 80,000 | 90,000 | 67,000 | 55,000 | | | | | | 52,000 | 60,000 |
| %eau ajoutée | | qsp100 | qsp100 | qsp100 | qsp100 | qsp100 | qsp100 | qsp100 | qsp100 | qsp10 0 | qsp100 | qsp100 |
| %Nacl max apporté par cocoamphoacetate | 7,6 | 0,00 | 0,00 | 0,78 | 0,00 | 0,00 | 0,00 | 0,00 | 0,00 | 0,00 | 0,00 | 0,00 |
| %Nacl max apporté par dans bétaine | 7,3 | 0,34 | 0,17 | 0,00 | 0,66 | 0,65 | 0,65 | 0,65 | 0,65 | 0,65 | 1,94 | 0,78 |
| %Nacl apporté par les tensio-actifs | | 0,34 | 0,17 | 0,78 | 0,66 | 0,65 | 0,65 | 0,65 | 0,65 | 0,65 | 1,94 | 0,78 |
| MA totale | | | | | | | | | | | | |
| %eau total | | | | | | | | | | | | |

**Tableau Il :**

| | HCSFE01 | HCSFE02 | HCSFB04 | HCSFB05 | HCSFB17 | HCSFB44 | HCSFB45 | HCSFB46 | HCSFB47 | HCSFE06 | HCSFE05 |
|---|---|---|---|---|---|---|---|---|---|---|---|
| Nom INCI | % MA | % MA | % MA | % MA | % MA | % MA | % MA | % MA | % MA | % MA | % MA |
| Ammonium lauryl sulfate | 4,68 | 2,34 | 9,25 | 9,25 | 7,68 | 7,68 | 7,68 | 7,68 | 7,68 | 9,45 | 10,88 |
| Sodium methyl cocoyl taurate | 0,72 | 0,00 | 0,00 | 0,00 | 0,00 | 0,00 | 0,00 | 0,00 | 0,00 | 0,00 | 0,00 |
| Sodium laurylsarcosinate | 0,00 | 0,45 | 0,00 | 0,00 | 0,00 | 0,00 | 0,00 | 0,00 | 0,00 | 0,00 | 0,00 |
| cocamidopropylbetaine | 1,61 | 0,81 | 0,00 | 3,19 | 3,12 | 3,12 | 3,12 | 3,12 | 3,12 | 9,31 | 3,75 |
| cocoamphoacetate | 0,00 | 0,00 | 3,30 | 0,00 | 0,00 | 0,00 | 0,00 | 0,00 | 0,00 | 0,00 | 0,00 |
| decylglucoside | 0,46 | 0,23 | 0,90 | 0,90 | 1,54 | 1,54 | 1,54 | 1,54 | 1,54 | 0,00 | 1,06 |
| glyceryl oleate | 0,15 | 0,08 | 0,00 | 0,00 | 0,00 | 0,00 | 0,00 | 0,00 | 0,00 | 0,00 | 0,36 |
| glyceryl oleate + cocoglucoside (LAMESOFT PO65 BASF) | 0,00 | 0,00 | 0,00 | 0,00 | 0,65 | 0,65 | 0,65 | 0,65 | 0,65 | 0,00 | 0,00 |
| acide citrique | 0,05 | 0,02 | 0,75 | 0,05 | 0,06 | 0,06 | 0,06 | 0,06 | 0,06 | 0,00 | 0,11 |
| parfum | 0,49 | 0,24 | 0,80 | 0,80 | 0,75 | 0,75 | 0,75 | 0,75 | 0,75 | 0,00 | 0,96 |
| gel aloe vera | 0,19 | 0,10 | 0,00 | 0,00 | 0,00 | 0,00 | 0,00 | 0,00 | 0,00 | 0,00 | 0,44 |
| extrait grenade | 0,00 | 0,00 | 0,00 | 0,00 | 0,00 | 0,00 | 0,00 | 0,00 | 0,00 | 0,00 | 0,20 |
| beurre Karité | 0,00 | 0,00 | 1,00 | 1,00 | 64,00 | 0,00 | 0,00 | 0,00 | 0,00 | 0,00 | 0,00 |
| cococaprylate caprate | 0,00 | 0,00 | 0,00 | 0,00 | 0,00 | 64,00 | 0,00 | 0,00 | 0,00 | 0,00 | 0,00 |
| isopropyl palmitate | 0,00 | 0,00 | 0,00 | 0,00 | 0,00 | 0,00 | 0,00 | 0,00 | 0,00 | 0,00 | 0,00 |
| undecane et tridecane | 0,00 | 0,00 | 0,00 | 0,00 | 0,00 | 0,00 | 64,00 | 0,00 | 0,00 | 0,00 | 0,00 |
| squalane | 0,00 | 0,00 | 0,00 | 0,00 | 0,00 | 0,00 | 0,00 | 64,00 | 0,00 | 0,00 | 0,00 |
| huile de colza | 0,00 | 0,00 | 0,00 | 0,00 | 0,00 | 0,00 | 0,00 | 0,00 | 56,70 | 0,00 | 0,00 |
| huile de coprah | 0,00 | 0,00 | 0,00 | 0,00 | 0,00 | 0,00 | 0,00 | 0,00 | 10,00 | 0,00 | 0,00 |
| huile tournesol | 80,00 | 90,00 | 67,00 | 55,00 | 0,00 | 0,00 | 0,00 | 0,00 | 0,00 | 52,00 | 60,00 |
| %eau ajoutée | | | | | | | | | | | |
| %Nacl max apporté par cocoamphoacetate | 0,00 | 0,00 | 0,78 | 0,00 | 0,00 | 0,00 | 0,00 | 0,00 | 0,00 | 0,00 | 0,00 |
| %Nacl max apporté par dans bétaine | 0,34 | 0,17 | 0,00 | 0,66 | 0,65 | 0,65 | 0,65 | 0,65 | 0,65 | 1,94 | 0,78 |
| %Nacl apporté par les tensio-actifs | 0,34 | 0,17 | 0,78 | 0,66 | 0,65 | 0,65 | 0,65 | 0,65 | 0,65 | 1,94 | 0,78 |
| MA totale | 7,62 | 3,90 | 13,45 | 13,33 | 12,99 | 12,99 | 12,99 | 12,99 | 12,99 | 18,76 | 16,05 |
| %eau total | 11,84 | 5,83 | 17,01 | 29,82 | 22,20 | 22,20 | 22,20 | 22,20 | 19,50 | 29,24 | 22,89 |

**Tableau III :**

| | HCSFE01 | HCSFE02 | HCSFB04 | HCSFB05 | HCSFB17 | HCSFB44 | HCSFB45 | HCSFB46 | HCSFB47 | HCSFE06 | HCSFE05 |
|---|---|---|---|---|---|---|---|---|---|---|---|
| Nom INCI | ratio | ratio | ratio | ratio | ratio | ratio | ratio | ratio | ratio | ratio | ratio |
| Ammonium lauryl sulfate | 0,61 | 0,60 | 0,69 | 0,69 | 0,59 | 0,59 | 0,59 | 0,59 | 0,59 | 0,50 | 0,68 |
| Sodium methyl cocoyl taurate | 0,09 | 0,00 | 0,00 | 0,00 | 0,00 | 0,00 | 0,00 | 0,00 | 0,00 | 0,00 | 0,00 |
| Sodium laurylsarcosinate | 0,00 | 0,12 | 0,00 | 0,00 | 0,00 | 0,00 | 0,00 | 0,00 | 0,00 | 0,00 | 0,00 |
| cocamidopropylbetaine | 0,21 | 0,21 | 0,00 | 0,24 | 0,24 | 0,24 | 0,24 | 0,24 | 0,24 | 0,50 | 0,23 |
| cocoamphoacetate | 0,00 | 0,00 | 0,25 | 0,00 | 0,00 | 0,00 | 0,00 | 0,00 | 0,00 | 0,00 | 0,00 |
| decylglucoside | 0,06 | 0,06 | 0,07 | 0,07 | 0,12 | 0,12 | 0,12 | 0,12 | 0,12 | 0,00 | 0,07 |
| glyceryl oleate | 0,02 | 0,02 | 0,00 | 0,00 | 0,00 | 0,00 | 0,00 | 0,00 | 0,00 | 0,00 | 0,02 |
| glyceryl oleate + cocoglucoside (LAMESOFT PO65 **BASF)** | 0,00 | 0,00 | 0,00 | 0,00 | 0,05 | 0,05 | 0,05 | 0,05 | 0,05 | 0,00 | 0,00 |

### Exemple 2 : ÉTUDE DE LA TOLÉRANCE CUTANÉE, DES QUALITÉS COSMÉTIQUES ET DE LA SATISFACTION GÉNÉRALE D'UNE EMULSION DE L'INVENTION « CREME DE DOUCHE KARITÉ »

Résumé des caractéristiques de l'étude :

| | |
|---|---|
| **Produit testé** | Crème de douche Karité -Soin Végétal Corps- Yves Rocher |
| | Mode d'application : Sous la douche et sur peau humide, appliquer sur l'ensemble du corps à la place du gel douche habituel. |
| **Caractéristique des volontaires** | Nombre de volontaires : 27 pour la tolérance, 25 pour le questionnaire d'appréciation |
| | Femmes de 40 à 75 ans (58 ans en moyenne) |
| | peaux extra-sèches (indice d'hydratation <25u.a.) |
| **Objectif** | Vérification de la tolérance cutanée, évaluation des qualités cosmétiques et de la satisfaction globale de 27 volontaires adultes, ayant donné leur consentement libre et éclairé, après application du produit testé 1 fois par jour minimum pendant 3 semaines sur le corps |
| **Méthode** | Comparaison des résultats des examens médicaux et des avis émis par les volontaires au début et à la fin de l'essai. |

Cette étude avait pour but :
(1) de vérifier la tolérance cutanée de Crème de douche Karité -Soin Végétal Corps-Yves Rocher appliquée dans des conditions normales, pendant 3 semaines, avec 27 volontaires adultes,
(2) d'évaluer les qualités cosmétiques et la satisfaction globale de la Crème de douche Karité -Soin Végétal Corps-Yves Rocher appliquée dans des conditions normales d'utilisation, pendant 3 semaines, avec 25 volontaires.

### PROTOCOLE DE L'ETUDE

Le produit étudié a été appliqué sur le corps 1 fois par jour minimum, pendant 3 semaines consécutives, par 27 volontaires adultes sains.

Ces applications ont été effectuées par les volontaires eux-mêmes, à son domicile et selon le protocole d'utilisation décrit par le promoteur.

Le produit étudié a été utilisé à la place du produit du même type que le volontaire utilise habituellement ou occasionnellement.

Déroulement de l'étude :
**A T0 :**
   - Les volontaires viennent sans avoir appliqué de produit cosmétique sur le corps le jour du rendez-vous.
   - Examen clinique et vérification des critères d'inclusion et de non-inclusion par le médecin.
   - Réalisation de mesures d'hydratation sur les bras et les demi-jambes des volontaires. Les sujets sont inclus dans l'étude si les valeurs obtenues sont strictement inférieures à 25 u.a. sur au moins une zone.
   - Les volontaires sélectionnés reçoivent un échantillon du produit à tester et un questionnaire d'évaluation du produit.
**De T0 à T 3 semaines :**
   - Les volontaires appliquent le produit testé à domicile et dans les conditions normales d'utilisation du produit, à la place de celui utilisé habituellement, et selon les indications mentionnées dans le questionnaire (fréquence et durée d'utilisation, zone et mode d'application).
   - Les volontaires évaluent les qualités cosmétiques du produit, la tolérance, les allégations et la satisfaction globale.
   - Les volontaires ne doivent pas utiliser de nouveaux produits cosmétiques (à l'exception de celui de l'étude).
   - Les volontaires ne doivent pas changer leurs habitudes d'utilisation de produits cosmétiques autres que le produit ayant les mêmes effets que le produit testé.
**A T3 semaines :**
   - Les volontaires viennent en consultation sans avoir appliqué de produit cosmétique sur le corps depuis 48 heures.
   - Examen clinique par le médecin, qui vérifie également le bon respect du protocole.
   - Les volontaires remettent le questionnaire d'évaluation et le produit testé.

### SUIVI DE L'ETUDE

27 volontaires adultes ont été recrutés.

Aucun sujet n'a été exclu des études. 2 volontaires n'ont pas rempli le questionnaire d'appréciation subjective.

Les caractéristiques des volontaires sont mentionnées dans le Tableau IV.

**Tableau IV :**

| **CARACTERISTIQUES DES VOLONTAIRES - 27 SUJETS** | |
|---|---|
| **Moyenne d'âge de l'étude:** | 58 ans |
| (âge min.: 40 ans; âge max.: 75 ans) | |
| **Type de peau** : | 100 % |
| - Extra sèche | |
| = index d'hydratation < 25 sur au moins 1 zone | |
| Historique de réaction cutanée au type de produit testé | 0% |
| Historique de réaction cutanée sue la zone d'application | 0% |

### RESULTATS ET DISCUSSION : Appréciation de la TOLERANCE CUTANEE

Pour 100 % des volontaires, les éventuels signes cliniques (sécheresse) présents à T0 ont diminué ou disparu, montrant une amélioration de l'état cutané ou une absence de changement. Ces volontaires n'ont relevé aucune réaction cutanée ou signe fonctionnel lié à l'utilisation de la Crème de douche Karité testée et ont jugé la tolérance excellente.

De plus, aucune réaction, ni irritation significative n'a été observée par le dermatologue après 3 semaines d'utilisation (J21) pour tous les volontaires examinés.

Par comparaison, en moyenne, 3,4% d'utilisateurs rapportent des réactions cutanées lors de l'utilisation de gels douche classiques.

Par comparaison, les réactions les plus souvent rapportées lors d'utilisation de gels douche classique sont une légères sécheresse et des picotements. Aucune de ces réactions n'a été observée lors de l'utilisation de la Crème de douche Karité.

### Résultats :

Il ressort de cette étude que les applications de la Crème de douche Karité -Soin Végétal Corps- Yves Rocher se sont avérées très bien tolérées sur le plan cutané pour les peaux extra-sèches.

L'évaluation des qualités cosmétiques révèle que le produit est bien apprécié.

Il obtient une note moyenne de 7,00/10.

Les volontaires ont principalement trouvé leur peau nourrie, douce, souple, confortable, lissée, lavée en douceur ; son équilibre est respecté. Les sensations de tiraillements sont minimisées, la peau ne présente pas de sensation d'inconfort. Le produit se rince facilement.

### Conclusion :

La Crème de douche Karité -Soin Végétal Corps- Yves Rocher, évaluée sous contrôle dermatologique, a été très bien tolérée.

Le produit est globalement bien apprécié au plan de l'efficacité perçue, et de ses qualités cosmétiques.

### Exemple 3 : ÉTUDE DE LA TOLÉRANCE CUTANÉE, DES QUALITÉS COSMÉTIQUES ET DE LA SATISFACTION GÉNÉRALE D'UNE EMULSION DE L'INVENTION « CREME DE DOUCHE AVOINE »

Résumé des caractéristiques de l'étude :

| | |
|---|---|
| **Produit testé** | Crème de douche Avoine -Soin Végétal Corps- Yves Rocher |
| | Mode d'application : Sous la douche et sur peau humide, appliquer sur l'ensemble du corps à la place du gel douche habituel. |
| **Caractéristique des volontaires** | Nombre de volontaires : 25 Femmes de 32 à 71 ans (57 ans en moyenne) |
| | Peaux très sèches |
| **Objectif** | Vérification de la tolérance cutanée, évaluation des qualités cosmétiques et de la satisfaction globale de 25 volontaires adultes, ayant donné leur consentement libre et éclairé, après application du produit testé 1 fois par jour minimum pendant 3 semaines sur le corps |
| **Méthode** | Comparaison des résultats des examens médicaux et des avis émis par les volontaires au début et à la fin de l'essai. |

Cette étude avait pour but :
(1) de vérifier la tolérance cutanée de Crème de douche Avoine -Soin Végétal Corps-Yves Rocher appliquée dans des conditions normales, pendant 3 semaines, avec 25 volontaires adultes,
(2) d'évaluer les qualités cosmétiques et la satisfaction globale de la Crème de douche Avoine -Soin Végétal Corps-Yves Rocher appliquée dans des conditions normales d'utilisation, pendant 3 semaines, avec 25 volontaires.

### PROTOCOLE DE L'ETUDE

Le produit étudié a été appliqué sur le corps 1 fois par jour minimum, pendant 3 semaines consécutives, par 25 volontaires adultes sains.

Ces applications ont été effectuées par les volontaires eux-mêmes, à son domicile et selon le protocole d'utilisation décrit par le promoteur.

Le produit étudié a été utilisé à la place du produit du même type que le volontaire utilise habituellement ou occasionnellement.

Déroulement de l'étude :
**A T0 :**
   - Les volontaires viennent sans avoir appliqué de produit cosmétique sur le corps le jour du rendez-vous.
   - Examen clinique et vérification des critères d'inclusion et de non-inclusion par le médecin.
   - Réalisation de mesures d'hydratation sur les bras et les demi-jambes des volontaires. Les sujets sont inclus dans l'étude si les valeurs obtenues sont strictement inférieures à 25 u.a. sur au moins une zone.
   - Les volontaires sélectionnés reçoivent un échantillon du produit à tester et un questionnaire d'évaluation du produit.
**De T0 à T 3 semaines :**
   - Les volontaires appliquent le produit testé à domicile et dans les conditions normales d'utilisation du produit, à la place de celui utilisé habituellement, et selon les indications mentionnées dans le questionnaire (fréquence et durée d'utilisation, zone et mode d'application).
   - Les volontaires évaluent les qualités cosmétiques du produit, la tolérance, les allégations et la satisfaction globale.
   - Les volontaires ne doivent pas utiliser de nouveaux produits cosmétiques (à l'exception de celui de l'étude).
   - Les volontaires ne doivent pas changer leurs habitudes d'utilisation de produits cosmétiques autres que le produit ayant les mêmes effets que le produit testé.
**A T3 semaines** :
   - Les volontaires viennent en consultation sans avoir appliqué de produit cosmétique sur le corps depuis 48 heures.
   - Examen clinique par le médecin, qui vérifie également le bon respect du protocole.
   - Les volontaires remettent le questionnaire d'évaluation et le produit testé.

### SUIVI DE L'ETUDE

25 volontaires adultes ont été recrutés pour le produit Olive.

Aucun sujet n'a été exclu des études.

Les caractéristiques des volontaires sont mentionnées dans le Tableau V.

**Tableau V :**

| **CARACTERISTIQUES DES VOLONTAIRES - 27 SUJETS** | |
|---|---|
| **Moyenne d'âge de l'étude:** | 57 ans |
| (âge min.: 32 ans; âge max.: 71 ans) | |
| **Type de peau :** | 100 % |
| Très sèche | |
| Historique de réaction cutanée au type de produit testé | 0% |
| Historique de réaction cutanée sue la zone d'application | 0% |

### RESULTATS ET DISCUSSION : Appréciation de la TOLERANCE CUTANEE

Pour 100 % des volontaires, les éventuels signes cliniques (sécheresse) présents à T0 ont diminué ou disparu, montrant une amélioration de l'état cutané ou une absence de changement. Ces volontaires n'ont relevé aucune réaction cutanée ou signe fonctionnel lié à l'utilisation de la Crème de douche Avoine testée et ont jugé la tolérance excellente.

De plus, aucune réaction, ni irritation significative n'a été observée par le dermatologue après 3 semaines d'utilisation (J21) pour tous les volontaires examinés.

Par comparaison, en moyenne, 3,4% d'utilisateurs rapportent des réactions cutanées lors de l'utilisation de gels douche classiques.

Par comparaison, les réactions les plus souvent rapportées ors d'utilisation de gels douche classique sont une légères sécheresse et des picotements. Aucune de ces réactions n'a été observée lors de l'utilisation de la Crème de douche Avoine.

### Résultats :

Il ressort de cette étude que les applications de la Crème de douche Avoine -Soin Végétal Corps- Yves Rocher se sont avérées très bien tolérées sur le plan cutané pour les peaux extra-sèches.

L'évaluation des qualités cosmétiques révèle que le produit est bien apprécié.

Il obtient une note moyenne de 7,32/10.

Les volontaires ont principalement trouvé leur peau nourrie, douce, souple, confortable, lissée, lavée en douceur ; son équilibre est respecté. Les sensations de tiraillements sont minimisées, la peau ne présente pas de sensation d'inconfort. Le produit se rince facilement.

### Conclusion :

La Crème de douche Avoine -Soin Végétal Corps- Yves Rocher, évaluée sous contrôle dermatologique, a été très bien tolérée.

Le produit est globalement bien apprécié au plan de l'efficacité perçue, et de ses qualités cosmétiques.

### Exemple 4: EVALUATION CHEZ L'HOMME DE L'EFFET NOURRISSANT DE LA CREME DE DOUCHE KARITE SOIN VEGETAL CORPS PAR CORNEOMETRIE

Résumé des caractéristiques de l'étude :

| | |
|---|---|
| **Produit testé** | Crème de douche Karité -Soin Végétal Corps- Yves Rocher |
| | Mode d'application : Application quotidienne pendant 21 jours sous la douche par légers massages sur l'ensemble du corps puis rincé. |
| **Caractéristique des volontaires** | Nombre de volontaires : 27 |
| | Age : 58 ans en moyenne (âge minimum : 40 ;âge maximum :74) |
| | Type de peau : Très sèche : 100% |
| | Zones de mesures : Avant-bras / Jambes |
| | Temps de mesures : T0 / T21 jours |
| **Objectif** | Evaluer par l'intermédiaire de mesures d'hydratation, l'effet nourrissant et protecteur vis-à-vis du dessèchement de la Crème de douche Karité Soin Végétal Corps. |
| **Méthode** | Une mesure diélectrique liée à l'état d'hydratation de la couche cornée est réalisée à T0 sur l'avant-bras et la jambe. Cette mesure est de nouveau réalisée sur les 2 zones après 21 jours d'utilisation du produit. |
| | L'évaluation de l'efficacité du produit se fait par comparaison des mesures avant/après, chaque volontaire étant son propre témoin. |
| | Une augmentation de l'hydratation, après l'utilisation longue durée d'un produit de douche contenant des ingrédients émollients, est le signe d'un effet nourrissant du produit et permet de conclure que la peau est protégée du dessèchement. |
| | Appareil: CORNEOMETRE CM825 (Courage et Khazaka) |

### Principe de l'étude/méthodologie

La mesure de l'hydratation de la peau est basée sur la méthode de la capacité diélectrique. Le Stratum Corneum (SC) se caractérise par une valeur moyenne diélectrique liée à la quantité d'eau contenue dans le SC.

La valeur du diélectrique de l'eau est très différente de celle de nombreuses autres substances contenues dans la peau, ce qui garantit une mesure très sélective.

Le capteur est constitué de deux électrodes métalliques en forme de peigne (or) qui induit un champ électrique à travers le stratum corneum et un courant électrique entre les deux électrodes.

L'appareil mesure la capacité correspondante, cette valeur est exprimée en unité arbitraire; plus elle est élevée, plus les couches superficielles de l'épiderme sont hydratées.

Une augmentation de l'hydratation, après l'utilisation à long terme d'un produit de douche contenant des ingrédients émollients, est révélatrice d'un effet nourrissant du produit et permet de conclure que la peau est protégée du dessèchement.

Les mesures sont effectuées avec un cornéomètre (COURAGE + KHAZAKA, Allemagne).

### Déroulement de l'étude

Les volontaires sont installés et laissés au repos pendant 5 minutes avant l'enregistrement dans une pièce climatisée avec une humidité contrôlée et une température de 22 +/- 2 ° C.

### A T0 :

- Les volontaires n'ont pas appliqué de produits cosmétiques au corps depuis 48 heures.
- Réalisation des mesures d'hydratation basées sur des repères anatomiques spécifiques sur les jambes et les avant-bras.
- Distribution du produit aux volontaires pour application dans la douche, par de légers massages sur tout le corps, puis rinçage.

### A T21 jours:

- Réalisation des mesures d'hydratation sur les volontaires n'ayant pas appliqué de produits cosmétiques sur le corps le jour du rendez-vous, sur les repères anatomiques définis à T0.

### Analyse et interprétation :

Les résultats obtenus ont été recueillis dans des manuels d'observation et / ou des formulaires de test, puis analysés et interprétés par l'enquêteur, en fonction des conditions normales d'utilisation et des effets recherchés par le promoteur.

Le calcul des valeurs moyennes est obtenu à partir des valeurs d'hydratation pour chaque volontaire avant et après l'application du produit d'essai, sur les jambes et sur les avant-bras.

Calcul de l'effet global du produit étudié en déterminant la variation en pourcentage par rapport à la mesure initiale, à partir des valeurs moyennes pour chaque zone. Les valeurs individuelles sont ajoutées.

Analyse et interprétation des résultats sur la base des données obtenues dans les conditions expérimentales.

### Analyse statistique (2 séries de données appariées):

La vérification est effectuée sur la question de savoir si les séries de données suivent ou non une loi normale conformément au test Shapiro-Wilk W (ce test s'applique à des nombres supérieurs ou égaux à 15, sinon on considère que la série de données suit une loi normale ):
- Si p> 5%, l'idée selon laquelle la série de données suit une loi normale au niveau de confiance de 95% est acceptée. Le test utilisé pour l'analyse statistique sera le moyen de comparaison du test t de Student.
- Si p <5%, l'idée selon laquelle les séries de données suivent une loi normale au niveau de confiance de 95% peut être rejetée. Le test utilisé pour l'analyse statistique sera le test de rang Wilcoxon signé comparant les médianes.

Le principe des tests de Student t et Wilcoxon signés consiste à poser une hypothèse nulle (H0) d'absence de différence entre l'effet avant et après le traitement (= 0) et une autre hypothèse H1 (notre hypothèse de recherche) d'une différence avant Et après traitement (<> 0).

Ensuite, la détermination est faite de la probabilité p d'observer une différence avant et après le traitement au moins aussi grande que celle qui a été observée si l'hypothèse nulle est vraie.
- Si p <5%, l'hypothèse nulle est rejetée. Ensuite, l'hypothèse alternative H1 d'une différence significative avant et après le traitement est acceptée.
- Si p> 5%, l'hypothèse nulle est acceptée. Les données n'ont pas permis de montrer une différence significative avant et après le traitement.

Logiciel utilisé : Statgraphics Centurion Version XV II.

### Résultats:

### Variations d'indices d'hydratation (unités arbitraires)

| | Jambes | | Avant bras | |
|---|---|---|---|---|
| Indice d'hydratation (unités arbitraires) | Moyenne | Ecart-type | Moyenne | Ecart-type |
| T0 | 18,1 | 5,98 | 25,3 | 8,47 |
| T21 jours | 25,6 | 6,23 | 28,3 | 6,61 |
| différence T0-T21 jours | 6,9 | 7,7 | 3,3 | 9,4 |
| différence % | 38% | | 13% | |
| p (significatif si < 0.05) | 7,09E-05 | | 3,24E-02 | |
| % de sujets répondants | 81% | | 74% | |

Dans les conditions de l'étude, après 21 jours d'application de la Crème de douche Karité Soin Végétal Corps, Yves Rocher, on observe une augmentation moyenne significative de l'indice d'hydratation de 38% au niveau des jambes et de 13% au niveau des avant-bras.

Conclusion: Dans les conditions de l'étude, la Crème de douche Karité Soin Végétal Corps nourrit la peau et la protège du dessèchement.

## Revendications

1. Emulsion huile dans l'eau moussante structurée en phases lamellaires comprenant :
a) au moins un tensio-actif anionique,
b) au moins un composé choisi parmi les tensio-actifs amphotères et les tensio-actifs non-ioniques, et
c) au moins une phase huile,
• dans laquelle la matière active totale desdits tensio-actifs est comprise de 4,0 à 30% en poids par rapport au poids total de ladite émulsion,
• dans laquelle le ratio de tensio-actif anionique est compris de 0,3 à 0,9, le ratio de tensio-actif amphotère est compris de 0,0 à 0,7, et le ratio de tensio-actif non-ionique est compris de 0,0 à 0,7, ledit ratio étant le rapport entre le pourcentage en poids de matière active dudit tensio-actif dans ladite émulsion et le pourcentage en poids de matière active totale desdits tensio-actifs dans ladite émulsion,
• dans laquelle ladite huile est comprise de 50 à 98 % en poids du poids total de l'émulsion, 50% étant exclu,
• le pH de la composition étant compris entre 4 et 8,
**caractérisée en ce que** :
• ledit au moins un tensio-actif anionique est choisi parmi :
o les alkyl sulfates,
o les alkyl éther sulfates choisis dans le groupe le sodium trideceth sulfate, l'ammonium laureth sulfate, le magnésium laureth sulfate et le triethanolamine laureth sulfate, le diethanolamine laurethsulfate, le monoethanolamine laurethsulfate, et leurs mélanges,
o les sulfonates,
o les phosphates et les alkyl phosphates,
o les iséthionates,
o les taurates,
o les alkyl sulfoacetates,
o les dérivés anioniques de protéines d'origine végétale,
o les dérivés des aminoacides,
o les acides carboxyliques et leurs sels,
o les sels d'acides gras issus de la saponification
o les dérivés d'acide lactique,
o les dérivés d'alkyl polyglucoside, et
o les sulfosuccinates choisis dans le groupe comprenant le disodium undecylenamido MEA- sulfosuccinate, le disodium lauryl sulfosuccinate, le disodium ricinoleamido MEA-sulfosuccinate, et leurs mélanges,
le composé b) comprenant un tensio-actif amphotère.

2. Emulsion selon la revendication 1, dans laquelle l'au moins une phase huile est choisie dans le groupe comprenant les huiles naturelles, les huiles de synthèse et les beurres, et leurs mélanges, et comprend éventuellement au moins un corps gras solide naturel ou synthétique.

3. Emulsion selon la revendication 2, dans laquelle :
• l'au moins une huile naturelle est choisie parmi les triglycérides naturels et les cires liquides, et leurs mélanges et l'au moins une huile de synthèse est choisie parmi les huiles d'origine naturelle ou pétrochimique, comme des alcools, des alcanes, des alcènes, des esters, des éthers, des huiles silicones et des huiles fluorées, et leurs mélanges,
• l'au moins un beurre est choisi parmi les corps gras pâteux naturels et les corps gras pâteux synthétiques,
• l'au moins un corps gras solide naturel ou synthétique est choisi parmi les cires naturelles, les alcools gras solides de 14 à 36 carbones, les acides gras solides de 14 à 36 carbones, les huiles végétales hydrogénées, les composés hydrocarbonés de 20 à 50 carbones.

4. Emulsion selon la revendication 3, dans laquelle :
• lesdits triglycérides naturels sont choisis parmi les huiles d'avocat, de sésame, de tournesol, de colza, de maïs, de soja, de carthame, de pépins de raisin, d'arachide, de ricin, de coco, de graines de coton, d'olive, de palmiste, de son de riz, d'amande douce, de canola, d'abricot, de noix et de germe de blé,
• lesdites cires liquides sont choisies parmi l'huile de jojoba et l'huile de lanoline,
• lesdits alcools sont choisis parmi les alcools gras liquides comme l'alcool isocétylique et l'octyldodecanol,
• lesdits alcanes liquides sont choisis parmi le C11 C12 isoalcane, des alcanes linéaires de C12 à C22, les huiles de paraffine, le C11 C12 isoparaffin, l'isohexadécane, l'isoéicosane, l'isododécane, le squalane et l'hémisqualane,
• lesdits alcènes est le squalène,
• lesdits esters sont choisis parmi le myristate d'isopropyle, le cococaprylate caprate, le palmitate d'éthylhexyle, les alkyl benzoate et le triglycéride C8 C10,
• ledit éther est le dicaprylyl éther,
• lesdites huiles silicone sont choisies parmi les huiles de silicone volatiles ou non volatiles comme les polydiméthylsiloxanes (PDMS), les cyclodiméthylsiloxanes et les cyclométhicones,
• ladite huile fluorée ou fluorosiliconée est le polyperfluoromethylisopropyl éther,
• lesdits corps gras pâteux naturels sont choisis parmi le beurre de karité, le beurre de cacao, le beurre de coco, l'huile de palme et la lanoline,
• lesdits corps gras pâteux synthétiques sont choisis parmi les dérivés de lanoline, les huiles hydrogénées, le myristyl myristate, le myristyl lactate, le bis-diglycéryl polyacyladipate-2, l'arachidyl propionate, les cires silicones, les élastomères silicone, le PEG-5 pentaérythrityl éther & PPG-5 pentaérythrityl éther & glycine soja stérols, les polyglycérides oléiques, linoléniques et hydroxystéariques et la vaseline.
• lesdites cires naturelles sont choisies parmi la cire d'abeille, la cire de carnauba, la cire de sumac, la cire de candelilla, et la lanoline
• lesdits alcools gras solides de 14 à 36 carbones sont choisis parmi les alcools myristique, cétylique, stéarique et béhénique,
• lesdits acides gras solides de 14 à 36 carbones sont choisis parmi l'acide myristique, cétylique, stéarique, hydroxystéarique et béhénique,
• lesdites huiles végétales hydrogénées sont choisies parmi l'huile de ricin hydrogénée et la cire de riz hydrogénée,
• lesdits composés hydrocarbonés de 20 à 50 carbones sont choisis parmi la cire de polyéthylène, la paraffine, l'ozokérite, la cire microcristalline.

5. Emulsion selon la revendication 4, dans laquelle :
- les dits alkyl sulfates sont choisis dans le groupe comprenant l'ammonium laurylsulfate, le sodium lauryl sulfate, le sodium coco sulfate, le potassium laurylsulfate, le magnésium laurylsulfate, le triethanolamine laurylsulfate, le diethanolamine laurylsulfate, le monoethanolamine laurylsulfate et leurs mélanges ;
- lesdits sulfonates sont choisis dans le groupe comprenant le sodium C14-16 olefin sulfonate, le sodium C14-17 sec-Alkyl sulfonate et le sodium xylene sulfonate et leurs mélanges ;
- lesdits phosphates et alkyl phosphates sont choisis dans le groupe comprenant le dicetyl phosphate, le C12-15 phosphate, le Potassium cetyl phosphate et le C9-15 alkyl phosphate et leurs mélanges ;
- lesdits iséthionates sont choisis dans le groupe comprenant le sodium cocoyl isethionate et le sodium Lauroyl Methyl Isethionate et leurs mélanges ;
- lesdits taurates sont choisis dans le groupe comprenant le sodium methyl cocoyl taurate et le sodium stearoyl taurate et leurs mélanges ;
- lesdits alkyl sulfoacetates est le sodium lauryl sulfoacetate et leurs mélanges ;
- lesdits dérivés anioniques de protéines d'origine végétale sont choisis parmi le sodium cocoyl apple amino acids, le sodium lauroyl oat amino acids, le sodium lauroyl wheat amino acids et le potassium lauroyl wheat amino acids et leurs mélanges ;
- les dérivés des aminoacides sont choisis parmi le sodium lauroyl glutamate, le sodium cocoyl glutamate, le potassium cocoyl glycinate, le sodium cocoyl alaninate et le sodium lauroyl sarcosinate et leurs mélanges ;
- les dits acides carboxyliques et leurs sels sont choisis dans le groupe comprenant le sodium lauryl glucose carboxylate et le sodium laureth-13 carboxylate et leurs mélanges ;
- les dits sels d'acides gras issus de la saponification sont choisis parmi les sels de l'acide laurique, l'acide myristique, l'acide palmitique et l'acide stéarique et leurs mélanges ;
- lesdits dérivés d'acide lactique sont choisis parmi le sodium stearoyl lactylate, le sodium isostearyl lactylate et le sodium cocoyl lactylate et leurs mélanges ;
- lesdits dérivés d'alkyl polyglucoside sont choisis parmi le sodium cocoglucoside tartrate, le disodium cocopolyglucose sulfosuccinate et le disodium cocopolyglucose citrate et leurs mélanges.

6. Emulsion selon l'une quelconque des revendications 1 à 4, dans laquelle ledit au moins un tensio-actif amphotère est choisi dans le groupe comprenant les N-alkylamidobétaines, les bétaines, les sultaines, les alkylpolyaminocarboxylates, les alkylamphoacétates, leurs dérivés, et les dérivés de la glycine.

7. Emulsion selon la revendication 6, dans laquelle :
- lesdits N-alkylamidobétaines sont choisies dans le groupe comprenant la cocamidopropyl bétaine et la lauramidopropyl bétaine et leurs mélanges ;
- lesdites bétaines sont choisis dans le groupe comprenant la coco bétaine et la lauryl bétaine et leurs mélanges ;
- lesdites sultaines est la cocamidopropyl hydroxysultaine,
- lesdits alkylpolyaminocarboxylates sont choisis dans le groupe comprenant le sodium Carboxymethyl Tallow Polypropylamine et le sodium Carboxymethyl Oleyl Polypropylamine et leurs mélanges ;
- lesdits alkylamphoacétates sont choisis dans le groupe comprenant le disodium cocoamphoacetate, le sodium cocoamphoacetate et le disodium lauroamphoacetate et leurs mélanges ;
- lesdits dérivés de la glycine est le cocoamphopolycarboxyglycinate.

8. Emulsion selon l'une quelconque des revendications 1 à 4, comprenant en outre un tensio-actif non-ionique choisi dans le groupe comprenant les alkypolyglucosides, les esters de glycéryle et d'acide gras, les esters de sucrose et d'acides gras, les esters de sucrose oxyalkylénés, les esters de glycérol oxyalkylénés, les esters d'acides gras et de polyéthylène glycol, les esters d'acide gras et de sorbitan, les alcools gras polyglycérolés et les dérivés de glucamine.

9. Emulsion selon la revendication 8, dans laquelle :
- lesdits alkypolyglucosides sont choisis dans le groupe comprenant le décyl glucoside, le lauryl glucoside, le caprylyl/capryl glucoside, et le coco glucoside, et leurs mélanges;
- lesdits esters de glycéryle et d'acide gras sont choisis parmi le glycéryl stéarate, le glycéryl ricinoleate, le glycéryl oléate, le glycéryl caprylate et le glycéryl caprate et leurs mélanges ;
- les esters de sucrose et d'acides gras sont choisis parmi le sucrose stéarate, le sucrose palmitate, le sucrose laurate et le sucrose distearate, et leurs mélanges ;
- les esters de sucrose oxyalkylénés sont choisis parmi le méthyl glucose caprate/caprylate/oleate, le PEG-120 Methyl glucose dioleate et le PEG-20 Methyl glucose sesquistearate, et leurs mélanges ;
- les esters de glycérol oxyalkylénés sont choisis parmi le PEG-7 Glyceryl cocoate, le PEG-80 Glyceryl cocoate, le PEG-30 Glyceryl cocoate et le PEG-200 hydrogenated glyceryl palmate, et leurs mélanges ;
- lesdits esters d'acides gras et de polyéthylène glycol sont choisis dans le groupe le PEG-8 stéarate, le PEG-20 stéarate, le PEG-40 stéarate, le
PEG-50 stéarate et le PEG-100 stéarate, et leurs mélanges ;
- lesdits esters d'acide gras et de sorbitan sont choisis dans le groupe comprenant le sorbitan palmitate, le sorbitan stéarate, le sorbitan tristéarate, le sorbitan oléate et le sorbitan trioléate, et leurs mélanges ;
- lesdits dérivés de glucamine sont choisis dans le groupe comprenant le capryloyl ou le caproyl méthyl glucamide, le lauroyl méthyl Glucamide, le lauroyl méthyl glucamide et le cocoyl méthyl glucamide, et leurs mélanges.

10. Emulsion selon la revendication 8 ou 9, dans laquelle ledit tensio-actif anionique est un alkyl sulfate, le tensio-actif amphotère est une N-alkylamidobétaïne ou un alkylamphoacétate, et le tensio-actif non-ionique est un alkypolyglucoside.

11. Emulsion selon la revendication 8 ou 9, dans laquelle ledit tensio-actif anionique est choisi parmi l'ammonium lauryl sulfate, le sodium methyl cocoyl taurate et le sodium laurylsarcosinate, le tensio-actif amphotère est la cocamidopropylbétaine ou le cocoamphoacétate de sodium, et le tensio-actif non-ionique est le décyl glucoside ou le glycéryl oléate.

12. Emulsion selon l'une quelconque des revendications précédentes, comprenant en outre du parfum dont le pourcentage en poids dans la composition peut aller de 0,00 à 10,000% en poids par rapport au poids total de ladite émulsion.

13. Emulsion selon l'une quelconque des revendications précédentes, ladite émulsion étant dépourvue de chlorure de sodium autre que celui pouvant être apporté par lesdits tensio-actifs.

14. Emulsion selon l'une quelconque des revendications précédentes, **caractérisée en ce qu'**elle est cosmétique ou dermatologique.

15. Emulsion selon l'une quelconque des revendications précédentes, ladite composition étant sous une forme choisie parmi une crème de douche, une crème nettoyante pour la peau du visage, une crème démaquillante, un shampooing et une crème lavante pour les mains.

16. Utilisation cosmétique d'une émulsion telle que définie dans l'une quelconque des revendications précédentes, pour nettoyer la peau et/ou les phanères.

17. Procédé de traitement cosmétique non thérapeutique comprenant l'application sur la peau et/ou des phanères d'une émulsion telle que définie dans l'une quelconque des revendications 1 à 15.

18. Procédé de traitement selon la revendication 17, comprenant en outre une étape de rinçage de ladite émulsion.

19. Procédé de préparation d'une émulsion huile dans l'eau moussante structurée en phases lamellaires telle que définie dans l'une quelconque des revendications 1 à 15, comprenant le mélange d'au moins une phase huile avec une composition de surfactant structurée en phases lamellaires, le pourcentage de ladite composition de surfactant étant compris de 2 à 50% en poids bornes incluses, par rapport au poids total de ladite émulsion, et le pourcentage de l'au moins une phase huile étant compris de 50 à 98% en poids, 50% étant exclu, par rapport au poids total de ladite émulsion,
ladite composition de surfactant comprenant :
a) au moins un tensio-actif anionique, et
b) au moins un composé choisi parmi les tensio-actifs amphotères et les tensio-actifs non-ioniques, et
• dans laquelle la matière active totale desdits tensio-actifs est comprise de 20 à 60% en poids par rapport au poids total de la dite composition,
• dans laquelle le ratio de tensio-actif anionique est compris de 0,3 à 0,9, et le ratio de tensio-actif amphotère est compris de 0,0 à 0,7 et le ratio de tensio-actif non-ionique est compris de 0,0 à 0,7, ledit ratio étant le rapport entre le pourcentage en poids de matière active dudit tensio-actif dans ladite composition et le pourcentage en poids de matière active totale desdits tensio-actifs dans ladite composition,
• le pH de la composition étant compris entre 4 et 8,
**caractérisée en ce que** :
• ledit au moins un tensio-actif anionique est choisi parmi :
o les alkyl sulfates,
o les alkyl éther sulfates choisis dans le groupe le sodium trideceth sulfate, l'ammonium laureth sulfate, le magnésium laureth sulfate et le triethanolamine laureth sulfate, le diethanolamine laurethsulfate, le monoethanolamine laurethsulfate, et leurs mélanges,
o les sulfonates,
o les phosphates et les alkyl phosphates,
o les iséthionates,
o les taurates,
o les alkyl sulfoacetates,
o les dérivés anioniques de protéines d'origine végétale,
o les dérivés des aminoacides,
o les acides carboxyliques et leurs sels,
o les sels d'acides gras issus de la saponification,
o les dérivés d'acide lactique,
o les dérivés d'alkyl polyglucoside, et
o les sulfosuccinates choisis dans le groupe comprenant le disodium undecylenamido MEA- sulfosuccinate, le disodium lauryl sulfosuccinate, le disodium ricinoleamido MEA-sulfosuccinate, et leurs mélanges,
le composé b) comprenant un tensio-actif amphotère.

## Patentansprüche

1. Schäumende Öl-in-Wasser-Emulsion, die in lamellaren Phasen strukturiert ist und Folgendes umfasst:
a) mindestens ein anionisches Tensid,
b) mindestens eine Verbindung, die unter den amphoteren Tensiden und den nichtionischen Tensiden ausgewählt ist, und
c) mindestens eine Ölphase,
• wobei der Gesamtwirkstoff der Tenside zwischen 4,0 und 30 Gew.-%, bezogen auf das Gesamtgewicht der Emulsion, beträgt,
• wobei das Verhältnis von anionischem Tensid 0,3 bis 0,9 beträgt, das Verhältnis von amphoterem Tensid 0,0 bis 0,7 beträgt und das Verhältnis von nichtionischem Tensid 0,0 bis 0,7 beträgt, wobei das Verhältnis das Verhältnis des Gewichtsprozentsatzes des aktiven Materials des Tensids in der Emulsion zu dem Gewichtsprozentsatz des gesamten aktiven Materials der Tenside in der Emulsion ist,
• wobei das Öl zwischen 50 und 98 Gew.-% des Gesamtgewichts der Emulsion ausmacht, wobei 50 % ausgeschlossen sind,
• der pH-Wert der Zusammensetzung zwischen 4 und 8 liegt,
**dadurch gekennzeichnet, dass**:
• das mindestens eine anionische Tensid ausgewählt ist unter:
o Alkylsulfaten,
∘ den Alkylethersulfaten, ausgewählt aus der Gruppe, die Natriumtridecethsulfat, Ammoniumlaurethsulfat, Magnesiumlaurethsulfat, Triethanolaminlaurethsulfat, Diethanolaminlaurethsulfat, Monoethanolaminlaurethsulfat und deren Mischungen umfasst
o Sulfonate,
o Phosphate und Alkylphosphate,
o Isethionate,
o Taurate,
o Alkylsulfoacetate,
o anionische Derivate von Proteinen pflanzlichen Ursprungs,
o Aminosäurederivate,
o Carbonsäuren und ihre Salze,
o Fettsäuresalze aus der Verseifung
o Milchsäurederivate,
o Alkylpolyglucosid-Derivate, und
o Sulfosuccinate, ausgewählt aus der Gruppe umfassend Dinatriumundecylenamido-MEA-Sulfosuccinat, Dinatriumlaurylsulfosuccinat, Dinatriumricinoleamido-MEA-Sulfosuccinat und Mischungen davon,
Verbindung b) umfassend ein amphoteres Tensid.

2. Emulsion nach Anspruch 1, wobei die mindestens eine Ölphase ausgewählt ist aus der Gruppe, die natürliche Öle, synthetische Öle und Butter sowie Mischungen davon umfasst, und gegebenenfalls mindestens ein natürliches oder synthetisches festes Fett enthält.

3. Emulsion nach Anspruch 2, wobei:
• das mindestens eine natürliche Öl ausgewählt ist aus natürlichen Triglyceriden und flüssigen Wachsen und deren Mischungen und das mindestens eine synthetische Öl ausgewählt ist aus Ölen natürlichen oder petrochemischen Ursprungs, wie Alkoholen, Alkanen, Alkenen, Estern, Ethern, Silikonölen und fluorierten Ölen und deren Mischungen,
• die mindestens eine Butter unter den natürlichen pastösen Fetten und den synthetischen pastösen Fetten ausgewählt wird,
• das mindestens eine natürliche oder synthetische feste Fett unter natürlichen Wachsen, festen Fettalkoholen mit 14 bis 36 Kohlenstoffatomen, festen Fettsäuren mit 14 bis 36 Kohlenstoffatomen, hydrierten Pflanzenölen und Kohlenwasserstoffverbindungen mit 20 bis 50 Kohlenstoffatomen ausgewählt ist.

4. Emulsion nach Anspruch 3, wobei:
• die natürlichen Triglyceride sind ausgewählt aus Avocado-, Sesam-, Sonnenblumen-, Raps-, Mais-, Soja-, Distel-, Traubenkern-, Erdnuss-, Rizinus-, Kokosnuss-, Baumwollsamen-, Oliven-, Palmkern-, Reiskleie-, Süßmandel-, Canola-, Aprikosen-, Walnuss- und Weizenkeimöl,
• die flüssigen Wachse aus Jojobaöl und Lanolinöl ausgewählt sind,
• die Alkohole unter den flüssigen Fettalkoholen wie Isocetylalkohol und Octyldodecanol ausgewählt sind,
• die flüssigen Alkane ausgewählt sind aus C11-C12-Isoalkan, linearen Alkanen von C12 bis C22, Paraffinölen, C11-C12-Isoparaffin, Isohexadecan, Isoeicosan, Isododecan, Squalan und Hemisqualan,
• die Alkenen Squalen sind,
• die Ester ausgewählt sind aus Isopropylmyristat, Cococaprylatcaprat, Ethylhexylpalmitat, Alkylbenzoat und C8-C10-Triglycerid,
• der Ether Dicaprylylether ist
• die Siliconöle aus flüchtigen oder nichtflüchtigen Siliconölen wie Polydimethylsiloxanen (PDMS), Cyclodimethylsiloxanen und Cyclomethiconen ausgewählt sind,
• das genannte fluorierte oder Fluorsilikonöl ist Polyperfluormethylisopropylether,
• die natürlichen pastösen Fette aus Sheabutter, Kakaobutter, Kokosnussbutter, Palmöl und Lanolin ausgewählt sind,
• die synthetischen pastösen Fette sind ausgewählt aus Lanolinderivaten, hydrierten Ölen, Myristylmyristat, Myristyllactat, Bis-Diglycerylpolyacyladipat-2, Arachidylpropionat, Silikonwachsen, Silikonelastomeren, PEG-5-Pentaerythritylether & PPG-5-Pentaerythritylether & Glycin-Sojasterolen, Öl-, Linolen- und Hydroxystearinpolyglyceriden und Vaseline,
• die natürlichen Wachse ausgewählt sind aus Bienenwachs, Carnaubawachs, Sumachwachs, Candelillawachs und Lanolin,
• die festen Fettalkohole mit 14 bis 36 Kohlenstoffatomen unter den Myristin-, Cetyl-, Stearin- und Behensäurealkoholen ausgewählt sind,
• die festen Fettsäuren mit 14 bis 36 Kohlenstoffatomen unter Myristinsäure, Cetylsäure, Stearinsäure, Hydroxystearinsäure und Behensäure ausgewählt sind,
• die hydrierten pflanzlichen Öle aus hydriertem Rizinusöl und hydriertem Reiswachs ausgewählt sind,
• die Kohlenwasserstoffverbindungen mit 20 bis 50 Kohlenstoffatomen unter Polyethylenwachs, Paraffin, Ozokerit und mikrokristallinem Wachs ausgewählt sind.

5. Emulsion nach Anspruch 4, wobei:
- die Alkylsulfate aus der Gruppe ausgewählt sind, die Ammoniumlaurylsulfat, Natriumlaurylsulfat, Natriumcocosulfat, Kalium laurylsulfat, Magnesium laurylsulfat, Triethanolaminlaurylsulfat, Diethanolaminlaurylsulfat, Monoethanolaminlaurylsulfat und Mischungen davon umfasst;
- die Sulfonate ausgewählt sind aus der Gruppe, die Natrium-C14-16-Olefinsulfonat, Natrium-C14-17-sek-Alkylsulfonat und Natrium-Xylolsulfonat und Mischungen davon umfasst;
- die Phosphate und Alkylphosphate aus der Gruppe ausgewählt sind, die Dicetylphosphat, C12-15-Phosphat, Kaliumcetylphosphat und C9-15-Alkylphosphat und deren Mischungen umfasst;
- die Isethionate aus der Gruppe ausgewählt sind, die Natriumcocoylisethionat und Natriumlauroylmethylisethionat und Mischungen davon umfasst;
- die Taurate aus der Gruppe ausgewählt sind, die Natriummethylcocoyltaurat und Natriumstearoyltaurat und Mischungen davon umfasst;
- bei den Alkylsulfoacetaten handelt es sich um Natriumlaurylsulfoacetat und Mischungen davon;
- die anionischen Derivate von Proteinen pflanzlichen Ursprungs unter Natriumcocoyl-Apfelaminosäuren, Natrium-Lauroyl-Haferaminosäuren, Natrium-Lauroyl-Weizenaminosäuren und Kalium-Lauroyl-Weizenaminosäuren sowie deren Mischungen ausgewählt sind;
- die Aminosäurederivate ausgewählt sind aus Natriumlauroylglutamat, Natriumcocoylglutamat, Kaliumcocoylglycinat, Natriumcocoylalaninat und Natriumlauroylsarkosinat sowie deren Mischungen;
- die Carbonsäuren und deren Salze aus der Gruppe ausgewählt sind, die Natriumlaurylglucosecarboxylat und Natriumlaureth-13-carboxylat und deren Mischungen umfasst;
- die Salze der Fettsäuren, die aus der Verseifung resultieren, ausgewählt sind aus den Salzen der Laurinsäure, der Myristinsäure, der Palmitinsäure und der Stearinsäure sowie deren Mischungen;
- die Milchsäurederivate ausgewählt sind aus Natriumstearoyllactylat, Natriumisostearyllactylat und Natriumcocoyllactylat sowie deren Mischungen;
- die Alkylpolyglucosidderivate ausgewählt sind aus Natriumcocoglucosidtartrat, Dinatriumcocopolyglucosesulfosuccinat und Dinatriumcocopolyglucosecitrat und deren Mischungen.

6. Emulsion nach einem der Ansprüche 1 bis 4, wobei das mindestens ein amphoteres Tensid ausgewählt ist aus der Gruppe bestehend aus N-Alkylamidobetainen, Betainen, Sultainen, Alkylpolyaminocarboxylaten, Alkylamphoacetaten, deren Derivaten und Glycinderivaten.

7. Emulsion nach Anspruch 6, wobei:
- die N-Alkylamidobetaine aus der Gruppe ausgewählt sind, die Cocamidopropylbetain und Lauramidopropylbetain und deren Mischungen umfasst;
- die Betaine ausgewählt sind aus der Gruppe, die Coco-Betain und Lauryl-Betain und deren Mischungen umfasst;
- das Sultain ist Cocamidopropylhydroxysultain;
- die Alkylpolyaminocarboxylate ausgewählt sind aus der Gruppe, die Natrium-Carboxymethyl-Talg-Polypropylamin und Natrium-Carboxymethyl-Oleyl-Polypropylamin und deren Mischungen umfasst;
- die Alkylamphoacetate aus der Gruppe ausgewählt sind, die Dinatriumcocoamphoacetat, Natriumcocoamphoacetat und Dinatriumlauroamphoacetat und deren Mischungen umfasst;
- das Glycinderivat ist Cocoamphopolycarboxyglycinat.

8. Emulsion nach einem der Ansprüche 1 bis 4, die zusätzlich ein nichtionisches Tensid enthält, das aus der Gruppe ausgewählt ist, die Alkylpolyglucoside, Glycerinfettsäureester, Saccharosefettsäureester, oxyalkylenierte Saccharoseester, oxyalkylenierte Glycerinester, Polyethylenglykolfettsäureester, Sorbitanfettsäureester, Polyglycerinfettalkohole und Glucaminderivate umfasst.

9. Emulsion nach Anspruch 8, wobei:
- die Alkypolyglucoside aus der Gruppe ausgewählt sind, die Decylglucosid, Laurylglucosid, Caprylyl-/Caprylglucosid und Coco-Glucosid sowie deren Mischungen umfasst;
- die Glycerylfettsäureester ausgewählt sind aus Glycerylstearat, Glycerylricinoleat, Glyceryloleat, Glycerylcaprylat und Glycerylcaprat und deren Mischungen;
- die Ester von Saccharose und Fettsäuren sind ausgewählt aus Saccharosestearat, Saccharopalmitat, Saccharolaurat und Saccharosedistearat sowie deren Mischungen;
- die oxyalkylierten Saccharoseester sind ausgewählt aus Methylglukose-Caprat/Caprylat-Oleat, PEG-120 Methylglukose-Dioleat und PEG-20 Methylglukose-Sesquistearat sowie deren Mischungen;
- die oxyalkylenierten Glycerinester sind ausgewählt aus PEG-7 Glycerylcocoat, PEG-80 Glycerylcocoat, PEG-30 Glycerylcocoat und PEG-200 hydriertes Glycerylpalmat und deren Mischungen;
- die Polyethylenglykolfettsäureester aus der Gruppe PEG-8 Stearat, PEG-20 Stearat, PEG-40 Stearat, PEG-50 Stearat und PEG-100 Stearat sowie Mischungen davon ausgewählt sind;
- die Sorbitanfettsäureester aus der Gruppe ausgewählt sind, die Sorbitanpalmitat, Sorbitanstearat, Sorbitantristearat, Sorbitanoleat und Sorbitantrioleat sowie Mischungen davon umfasst;
- Lauroylmethylglucamid, Lauroylmethylglucamid und Cocoylmethylglucamid sowie deren Mischungen. Diese Glucaminderivate sind ausgewählt aus der Gruppe, die Capryloyl- oder Caproylmethylglucamid, Lauroylmethylglucamid, Lauroylmethylglucamid und Cocoylmethylglucamid sowie deren Mischungen umfasst.

10. Emulsion nach Anspruch 8 oder 9, wobei das anionische Tensid ein Alkylsulfat ist, das amphotere Tensid ein N-Alkylamidobetain oder ein Alkylamphoacetat ist und das nichtionische Tensid ein Alkypolyglucosid ist.

11. Emulsion nach Anspruch 8 oder 9, wobei das anionische Tensid ausgewählt ist aus Ammoniumlaurylsulfat, Natriummethylcocoyltaurat und Natriumlaurylsarkosinat, das amphotere Tensid Cocamidopropylbetain oder Natriumcocoamphoacetat ist und das nichtionische Tensid Decylglucosid oder Glycerinoleat ist.

12. Emulsion nach einem der vorhergehenden Ansprüche, die zusätzlich Parfüm enthält, dessen Gewichtsprozent in der Zusammensetzung zwischen 0,00 und 10,000 Gew.-%, bezogen auf das Gesamtgewicht der Emulsion, liegen kann.

13. Emulsion nach einem der vorhergehenden Ansprüche, wobei die Emulsion kein anderes Natriumchlorid enthält als das, das von den Tensiden geliefert werden kann.

14. Emulsion nach einem der vorangehenden Ansprüche, **dadurch gekennzeichnet, dass** sie kosmetisch oder dermatologisch ist.

15. Emulsion nach einem der vorhergehenden Ansprüche, wobei die Zusammensetzung in einer Form vorliegt, die ausgewählt ist aus einer Duschcreme, einer Reinigungscreme für die Gesichtshaut, einer Abschminkcreme, einem Shampoo und einer Handwaschcreme.

16. Kosmetische Verwendung einer Emulsion nach einem der vorhergehenden Ansprüche zur Reinigung der Haut und/oder der Hautanhangsgebilde.

17. Verfahren zur nicht-therapeutischen kosmetischen Behandlung, dass das Auftragen einer Emulsion nach einem der Ansprüche 1 bis 15 auf die Haut und/oder die Hautanhangsgebilde umfasst.

18. Behandlungsverfahren nach Anspruch 17, das ferner einen Schritt des Spülens der Emulsion umfasst.

19. Verfahren zur Herstellung einer schäumenden Öl-in-Wasser-Emulsion mit Lamellenphasenstruktur nach einem der Ansprüche 1 bis 15, bei dem man mindestens eine Ölphase mit einer Tensidzusammensetzung mit Lamellenphasenstruktur mischt, wobei der prozentuale Anteil der Tensidzusammensetzung 2 bis einschließlich 50 Gew.-% beträgt, bezogen auf das Gesamtgewicht der Emulsion, und der prozentuale Anteil der mindestens einen Ölphase 50 bis 98 Gew.-% beträgt, wobei 50 % ausgeschlossen sind, bezogen auf das Gesamtgewicht der Emulsion, wobei die Tensidzusammensetzung umfasst:
a) mindestens ein anionisches Tensid und
b) mindestens eine Verbindung, die unter den amphoteren Tensiden und den nichtionischen Tensiden ausgewählt ist, und
• wobei der Gesamtwirkstoff der Tenside 20 bis 60 Gew.-%, bezogen auf das Gesamtgewicht der Zusammensetzung, ausmacht,
• wobei das Verhältnis des anionischen Tensids 0,3 bis 0,9 beträgt und das Verhältnis des amphoteren Tensids 0,0 bis 0,7 beträgt und das Verhältnis des nichtionischen Tensids 0,0 bis 0,7 beträgt, wobei das Verhältnis das Verhältnis des Gewichtsprozentsatzes des aktiven Materials des Tensids in der Zusammensetzung zu dem Gewichtsprozentsatz des gesamten aktiven Materials der Tenside in der Zusammensetzung ist,
• der pH-Wert der Zusammensetzung zwischen 4 und 8 liegt,
**dadurch gekennzeichnet, dass** :
• dass mindestens eine anionische Tensid ausgewählt ist unter:
∘ alkyl sulfates,
∘ Alkylethersulfate, ausgewählt aus der Gruppe, die Natriumtridecethsulfat, Ammoniumlaurethsulfat, Magnesiumlaurethsulfat und Triethanolaminlaurethsulfat, Diethanolaminlaurethsulfat, Monoethanolaminlaurethsulfat und deren Mischungen umfasst,
∘ Sulfonate,
∘ Phosphate und Alkylphosphate,
∘ Isethionate,
∘ Taurate,
∘ Alkylsulfoacetate,
∘ anionische Derivate von Proteinen pflanzlichen Ursprungs,
∘ Aminosäurederivate,
∘ Carbonsäuren und ihre Salze,
o Fettsäuresalze aus der Verseifung,
o Milchsäurederivate,
o Alkylpolyglucosidderivate und
o Sulfosuccinate, ausgewählt aus der Gruppe bestehend aus Dinatriumundecylenamido-MEA-Sulfosuccinat, Dinatriumlaurylsulfosuccinat, Dinatriumricinoleamido-MEA-Sulfosuccinat und Mischungen davon,
Verbindung b), die ein amphoteres Tensid enthält.

## Claims

1. Foaming oil-in-water emulsion structured in lamellar phases comprising :
a) at least one anionic surfactant,
b) at least one compound chosen from amphoteric surfactants and non-ionic surfactants, and
c) at least one oil phase,
• wherein the total active matter of the said surfactants is between 4.0 and 30% by weight relative to the total weight of the said emulsion,
• wherein the ratio of anionic surfactant is from 0.3 to 0.9, the ratio of amphoteric surfactant is from 0.0 to 0.7, and the ratio of non-ionic surfactant is from 0.0 to 0.7, said ratio being the ratio of the percentage by weight of active material of said surfactant in said emulsion to the percentage by weight of total active material of said surfactants in said emulsion,
• wherein said oil is between 50 and 98% by weight of the total weight of the emulsion, 50% being excluded,
• the pH of the composition being between 4 and 8,
**characterised in that**:
• said at least one anionic surfactant is chosen from :
∘ alkyl sulfates,
∘ alkyl ether sulfates selected from the group comprising sodium trideceth sulfate, ammonium laureth sulfate, magnesium laureth sulfate and triethanolamine laureth sulfate, diethanolamine laureth sulfate, monoethanolamine laureth sulfate, and mixtures thereof
∘ sulfonates,
∘ phosphates and alkyl phosphates,
∘ isethionates,
∘ taurates,
∘ alkyl sulfoacetates,
∘ anionic derivatives of proteins of plant origin,
∘ amino acid derivatives,
∘ carboxylic acids and their salts,
∘ fatty acid salts from saponification
∘ lactic acid derivatives,
∘ alkyl polyglucoside derivatives, and
∘ sulfosuccinates selected from the group comprising disodium undecylenamido MEA sulfosuccinate, disodium lauryl sulfosuccinate, disodium ricinoleamido MEA sulfosuccinate, and mixtures thereof,
compound b) comprising an amphoteric surfactant.

2. Emulsion according to claim 1, wherein the at least one oil phase is chosen from the group comprising natural oils, synthetic oils and butters, and mixtures thereof, and optionally comprises at least one natural or synthetic solid fat.

3. Emulsion according to claim 2, wherein :
• the at least one natural oil is chosen from natural triglycerides and liquid waxes, and mixtures thereof, and the at least one synthetic oil is chosen from oils of natural or petrochemical origin, such as alcohols, alkanes, alkenes, esters, ethers, silicone oils and fluorinated oils, and mixtures thereof,
• the at least one butter is chosen from natural pasty fats and synthetic pasty fats,
• the at least one natural or synthetic solid fat is chosen from natural waxes, solid fatty alcohols with 14 to 36 carbons, solid fatty acids with 14 to 36 carbons, hydrogenated vegetable oils and hydrocarbon compounds with 20 to 50 carbons.

4. Emulsion according to claim 3, wherein :
• said natural triglycerides are selected from avocado, sesame, sunflower, rapeseed, maize, soya, safflower, grapeseed, peanut, castor, coconut, cottonseed, olive, palm kernel, rice bran, sweet almond, canola, apricot, walnut and wheat germ oils,
• said liquid waxes are chosen from jojoba oil and lanolin oil,
• said alcohols are chosen from liquid fatty alcohols such as isocetyl alcohol and octyldodecanol,
• said liquid alkanes are selected from C11 C12 isoalkane, linear alkanes from C12 to C22, paraffin oils, C11 C12 isoparaffin, isohexadecane, isoeicosane, isododecane, squalane and hemisqualane,
• said alkenes is squalene,
• said esters are chosen from isopropyl myristate, cococaprylate caprate, ethylhexyl palmitate, alkyl benzoate and C8 C10 triglyceride,
• said ether is dicaprylyl ether
• said silicone oils are selected from volatile or non-volatile silicone oils such as polydimethylsiloxanes (PDMS), cyclodimethylsiloxanes and cyclomethicones,
• said fluorinated or fluorosilicone oil is polyperfluoromethylisopropyl ether,
• said natural pasty fats are chosen from shea butter, cocoa butter, coconut butter, palm oil and lanolin,
• said synthetic pasty fats are chosen from lanolin derivatives, hydrogenated oils, myristyl myristate, myristyl lactate, bis-diglyceryl polyacyladipate-2, arachidyl propionate, silicone waxes, silicone elastomers, PEG-5 pentaerythrityl ether & PPG-5 pentaerythrityl ether & glycine soya sterols, oleic, linolenic and hydroxystearic polyglycerides and petroleum jelly,
• the said natural waxes are chosen from beeswax, carnauba wax, sumac wax, candelilla wax and lanolin,
• said solid fatty alcohols with 14 to 36 carbons are chosen from myristic, cetyl, stearic and behenic alcohols,
• said solid fatty acids with 14 to 36 carbons are chosen from myristic, cetyl, stearic, hydroxystearic and behenic acid,
• said hydrogenated vegetable oils are selected from hydrogenated castor oil and hydrogenated rice wax,
• the said hydrocarbon compounds with 20 to 50 carbons are chosen from polyethylene wax, paraffin, ozokerite and microcrystalline wax.

5. Emulsion according to claim 4, wherein :
- said alkyl sulfates are selected from the group comprising ammonium lauryl sulfate, sodium lauryl sulfate, sodium coco sulfate, potassium lauryl sulfate, magnesium lauryl sulfate, triethanolamine lauryl sulfate, diethanolamine lauryl sulfate, monoethanolamine lauryl sulfate and mixtures thereof;
- said sulfonates are selected from the group comprising sodium C14-16 olefin sulfonate, sodium C14-17 sec-Alkyl sulfonate and sodium xylene sulfonate and mixtures thereof;
- said phosphates and alkyl phosphates are selected from the group comprising dicetyl phosphate, C12-15 phosphate, potassium cetyl phosphate and C9-15 alkyl phosphate and mixtures thereof;
- said isethionates are selected from the group comprising sodium cocoyl isethionate and sodium lauroyl methyl isethionate and mixtures thereof;
- said taurates are selected from the group comprising sodium methyl cocoyl taurate and sodium stearoyl taurate and mixtures thereof;
- said alkyl sulfoacetates is sodium lauryl sulfoacetate and mixtures thereof;
- said anionic derivatives of proteins of plant origin are chosen from sodium cocoyl apple amino acids, sodium lauroyl oat amino acids, sodium lauroyl wheat amino acids and potassium lauroyl wheat amino acids and mixtures thereof;
- the amino acid derivatives are chosen from sodium lauroyl glutamate, sodium cocoyl glutamate, potassium cocoyl glycinate, sodium cocoyl alaninate and sodium lauroyl sarcosinate, and mixtures thereof;
- said carboxylic acids and salts thereof are selected from the group comprising sodium lauryl glucose carboxylate and sodium laureth-13 carboxylate and mixtures thereof;
- the said salts of fatty acids resulting from saponification are chosen from salts of lauric acid, myristic acid, palmitic acid and stearic acid and mixtures thereof;
- the said lactic acid derivatives are chosen from sodium stearoyl lactylate, sodium isostearyl lactylate and sodium cocoyl lactylate and mixtures thereof;
- said alkyl polyglucoside derivatives are chosen from sodium cocoglucoside tartrate, disodium cocopolyglucose sulfosuccinate and disodium cocopolyglucose citrate and mixtures thereof.

6. Emulsion according to anyone of claims 1 to 4, wherein said at least one amphoteric surfactant is chosen from the group comprising N-alkylamidobetaines, betaines, sultaines, alkylpolyaminocarboxylates, alkylamphoacetates, their derivatives, and glycine derivatives.

7. Emulsion according to claim 6, wherein :
- said N-alkylamidobetaines are selected from the group comprising cocamidopropyl betaine and lauramidopropyl betaine and mixtures thereof;
- said betaines are selected from the group comprising coco betaine and lauryl betaine and mixtures thereof;
- said sultains is cocamidopropyl hydroxysultaine ;
- said alkylpolyaminocarboxylates are selected from the group comprising sodium Carboxymethyl Tallow Polypropylamine and sodium Carboxymethyl Oleyl Polypropylamine and mixtures thereof;
- said alkylamphoacetates are selected from the group comprising disodium cocoamphoacetate, sodium cocoamphoacetate and disodium lauroamphoacetate and mixtures thereof;
- said glycine derivatives is cocoamphopolycarboxyglycinate.

8. Emulsion according to anyone of claims 1 to 4, additionally comprising a nonionic surfactant chosen from the group comprising alkylpolyglucosides, glyceryl fatty acid esters, sucrose fatty acid esters, oxyalkylenated sucrose esters, oxyalkylenated glycerol esters, polyethylene glycol fatty acid esters, sorbitan fatty acid esters, polyglycerol fatty alcohols and glucamine derivatives.

9. Emulsion according to claim 8, wherein :
- said alkypolyglucosides are selected from the group comprising decyl glucoside, lauryl glucoside, caprylyl/capryl glucoside, and coco glucoside, and mixtures thereof;
- said glyceryl fatty acid esters are chosen from glyceryl stearate, glyceryl ricinoleate, glyceryl oleate, glyceryl caprylate and glyceryl caprate and mixtures thereof;
- the sucrose and fatty acid esters are chosen from sucrose stearate, sucrose palmitate, sucrose laurate and sucrose distearate, and mixtures thereof;
- the oxyalkylenated sucrose esters are chosen from methyl glucose caprate/caprylate/oleate, PEG-120 methyl glucose dioleate and PEG-20 methyl glucose sesquistearate, and mixtures thereof;
- the oxyalkylenated glycerol esters are chosen from PEG-7 Glyceryl cocoate, PEG-80 Glyceryl cocoate, PEG-30 Glyceryl cocoate and PEG-200 hydrogenated glyceryl palmate, and mixtures thereof;
- said polyethylene glycol fatty acid esters are selected from the group PEG-8 stearate, PEG-20 stearate, PEG-40 stearate, PEG-50 stearate and PEG-100 stearate, and mixtures thereof;
- said sorbitan fatty acid esters are selected from the group comprising sorbitan palmitate, sorbitan stearate, sorbitan tristearate, sorbitan oleate and sorbitan trioleate, and mixtures thereof;
- , le lauroyl méthyl Glucamide, le lauroyl méthyl glucamide et le cocoyl méthyl glucamide, et leurs mélanges. said glucamine derivatives are selected from the group comprising capryloyl or caproyl methyl glucamide, le lauroyl methyl glucamide, lauroyl methyl glucamide and cocoyl methyl glucamide, and mixtures thereof.

10. Emulsion according to claim 8 or 9, wherein said anionic surfactant is an alkyl sulfate, the amphoteric surfactant is an N-alkylamidobetaine or an alkylamphoacetate, and the non-ionic surfactant is an alkypolyglucoside.

11. Emulsion according to claim 8 or 9, wherein said anionic surfactant is chosen from ammonium lauryl sulfate, sodium methyl cocoyl taurate and sodium lauryl sarcosinate, the amphoteric surfactant is cocamidopropylbetaine or sodium cocoamphoacetate, and the nonionic surfactant is decyl glucoside or glyceryl oleate.

12. Emulsion according to anyone of the preceding claims, additionally comprising perfume, the percentage by weight of which in the composition may range from 0.00 to 10.000% by weight relative to the total weight of the said emulsion.

13. Emulsion according to anyone of the preceding claims, said emulsion being devoid of sodium chloride other than the one that may be provided by the said surfactants.

14. Emulsion according to anyone of the preceding claims, **characterized in that** it is cosmetic or dermatological.

15. Emulsion according to anyone of the preceding claims, said composition being in a form chosen from a shower cream, a cleansing cream for facial skin, a make-up removal cream, a shampoo and a hand washing cream.

16. Cosmetic use of an emulsion as defined in anyone of the preceding claims, for cleansing the skin and/or the appendages.

17. Method of non-therapeutic cosmetic treatment comprising the application to the skin and/or the appendages of an emulsion as defined in anyone of claims 1 to 15.

18. Treatment method according to claim 17, further comprising a step of rinsing said emulsion.

19. Process for preparing a lamellar-phase-structured foaming oil-in-water emulsion as defined in anyone of claims 1 to 15, comprising mixing at least one oil phase with a lamellar-phase-structured surfactant composition, the percentage of the said surfactant composition being from 2 to 50% by weight, inclusive, based on the total weight of the said emulsion, and the percentage of the at least one oil phase being from 50 to 98% by weight, 50% being excluded, based on the total weight of the said emulsion, said surfactant composition comprising :
a) at least one anionic surfactant, and
b) at least one compound chosen from amphoteric surfactants and non-ionic surfactants, and
• wherein the total active matter of the said surfactants is from 20 to 60% by weight relative to the total weight of the said composition,
• wherein the ratio of anionic surfactant is from 0.3 to 0.9, and the ratio of amphoteric surfactant is from 0.0 to 0.7 and the ratio of non-ionic surfactant is from 0.0 to 0.7, said ratio being the ratio of the percentage by weight of active material of said surfactant in said composition to the percentage by weight of total active material of said surfactants in said composition,
• the pH of the composition being between 4 and 8,
**characterised in that** :
• said at least one anionic surfactant is chosen from:
∘ alkyl sulfates,
∘ alkyl ether sulfates chosen in the group comprising sodium trideceth sulfate, ammonium laureth sulfate, magnesium laureth sulfate and triethanolamine laureth sulfate, diethanolamine laurethsulfate, monoethanolamine laurethsulfate, and mixtures thereof,
∘ sulfonates,
∘ phosphates and alkyl phosphates,
∘ isethionates,
∘ taurates,
∘ alkyl sulfoacetates,
∘ anionic derivatives of proteins of plant origin,
∘ amino acid derivatives,
∘ carboxylic acids and their salts,
∘ fatty acid salts from saponification,
∘ lactic acid derivatives,
∘ alkyl polyglucoside derivatives, and
∘ sulfosuccinates selected from the group comprising disodium undecylenamido MEA sulfosuccinate, disodium lauryl sulfosuccinate, disodium ricinoleamido MEA sulfosuccinate, and mixtures thereof,
compound b) comprising an amphoteric surfactant.
